# EUROPEAN PATENT SPECIFICATION

(11) **EP 2 101 848 B1**
(45) Date of publication and mention of the grant of the patent: **16.04.2014**
(21) Application number: 07856741.9
(22) Date of filing: 14.12.2007
(51) Int. Cl.: A61M 15/00, A61M 11/00, A61M 16/00

(54) **Improvements in and relating to metered dose inhalers**
Verbesserungen von und im Zusammenhang mit Dosierinhalatoren
Améliorations des inhalateurs doseurs et relatives à ceux-ci

(30) Priority: 19.12.2006 GB 0625303
(43) Date of publication of application: 23.09.2009
(73) Proprietor: Jagotec AG, 4132 Mutenz (CH)
(72) Inventor: ZANGERLE, Wolfgang, 507 Mellingen (CH)
(74) Representative: Mintz Levin Cohn Ferris Glovsky and Popeo LLP
(86) International application number: PCT/EP2007/011003
(87) International publication number: WO 2008/074441

(56) References cited:
- WO-A-01/21238
- WO-A-97/20589
- WO-A-98/30262
- WO-A-99/51291
- DE-A1-102004 041 524
- DE-A1-102006 029 753
- FR-A- 2 701 653

## Description

The present invention relates to an inhaler for dispensing a dose of an active agent to the respiratory tract of a patient. The invention further encompasses a kit of parts for assembling an inhaler in accordance with the invention.

Metered dose inhalers for dispensing measured doses of active agent, typically in spray or powder form, are well known in the art. Such devices typically include a reservoir of active agent and an airway for delivering a dose of the agent to a mouthpiece for inhalation by a patient. "Active" inhalers further include a compressed air source which can be operated to force pressurised air through the device, aerosolising the active agent and delivering a dose to the mouthpiece and thence into the respiratory tract of a patient. "Passive" devices rely instead primarily upon suction applied by the patient to the mouthpiece to draw a dose of the active agent from the reservoir to the mouthpiece and then into the lungs. Some devices may have both "active" and "passive" functions or modes.

The reservoir, airways and, in the case of active devices, pressurised air source, are usually accommodated within a housing. Typically, the housing is manufactured from a plastics material in two or more pieces which are assembled and welded together during the manufacturing process to create the finalised product. By welding together the constituent parts of the housing, an air-tight connection is usually ensured; however the welding process is expensive and is occasionally unreliable, depending largely upon the nature of the plastics material employed. In some cases, the welding process may not result in the formation of a perfectly air-tight connection.

Devices which may be manufactured without welding are known in the art. Such devices include a housing which is formed from two or more parts that are connected together by way of a mechanical fixing, such as a click-fit fixing. Such devices are however usually found to be imperfectly air-tight, as air used to draw or propel active agent through the device escapes through and around the fixing, resulting in low efficiency of operation. In the case of a passive inhaler, this greatly increases the level of suction that is required to operate the device effectively. Thus, patients with low or impaired suction ability, such as young children or those suffering from conditions such as chronic obstructive pulmonary disease, may be unable to make use of the inhaler.

An inhaler of the prior art is disclosed in WO 01/21238.

There is therefore an outstanding need in the art for a metered dose inhaler which may be manufactured without welding, but which is air-tight and hence efficient in operation. In particular, there is a need for a passive inhaler which may be manufactured without welding and which may be effectively operated by patients with reduced or compromised respiratory capability.

The present invention is defined by the appended claims.

According to a first aspect of the present invention therefore, there is provided an inhaler for dispensing a dose of an active agent to the respiratory tract of a patient, which inhaler comprises a housing defined by an upper part and a lower part and a mouthpiece which is attached or is adapted to be attached to said housing; wherein said housing includes storage means for storing an active agent and an airway for delivering said dose from the storage means to the mouthpiece in a stream of air or other gas for administration to a patient, characterised in that said upper part and said lower part of the housing are joined together in a substantially airtight fashion by means of a mechanical fixing, such that a gas flow rate of no more than about 45 litres/minute, preferably no more than about 40 litres/minute, is required in order to propel a dose of said active agent from the reservoir to the mouthpiece for administration to the patient.

Suitably, the inhaler according to the first aspect of the present invention may be constructed such that a gas flow rate in the range of about 30 to about 40 litres/minute is required in order to propel a dose of said active agent from the reservoir to the mouthpiece for administration to the patient

The minimum gas flow rate needed to actuate the device can be measured using a Dosage Unit Sampling Apparatus (DUSA). DUSA is normally employed to measure doses of powder emitted from devices and the variance thereof. However, by adjusting the flow control unit to increase the flow rate by intervals, e.g. intervals of 5 litre/min, one is able to determine the flow rate required to actuate the dosing mechanism as described more fully herein. The measurement methodology and the apparatus therefor are well known in the art, and are described in the United States Pharmacopoeia Chapter <601>, or in the inhalants monograph of the European Pharmacopoeia.

In a typical procedure flow control modules are equipped with Draeger Volumeter, Inhalation Test Box and a DUSA. Air flow rates of 30 L/min, 35 L/min, 40 L/min, and 45 L/min, are pre-adjusted to each individual test set up. If flow rates >45 L/min were necessary then the flow rate of a flow control module can be re-adjusted. Each inhalation device tested is shaken, opened and inserted into the first test set-up with the air flow rate adjusted to 30 L/min. A simulated inhalation is actuated for duration of 8 seconds, to reach a total simulated inspiration volume of 4 L. If the actuation of the device and release of the dose was observed, the device was removed from the apparatus. If an inhaler device fails to actuate at the lowest air flow rate, the procedure can be repeated once at the same flow rate. If an inhaler device fails to actuate a second time, actuation of the device can be attempted at 35 L/min, using the test setup with the next incrementally increased pre-set flow rate. The test can be repeated with increasing the flow rate in steps of 5 L/min, until successful actuation takes place. The result was documented with the specific actuation flow rate in the work sheet.

According to a second aspect of the present invention, there is provided an inhaler for multiple dosed administration of a pharmacological dry powder, comprising a housing including a lower part and an upper part; a mouthpiece attached to an end of said housing, said mouthpiece including a channel extending therethrough; a protective cap covering said mouthpiece, said protective cap being movable between a closed position and an open position; a medicament reservoir located inside said housing, said medicament reservoir containing dry powder and including a funnel outlet; a movable shutter positioned inside said housing, said shutter including a closure part which protrudes into said channel of said mouthpiece; a movable dosing slide located inside said housing, said dosing slide being movable between a receiving position and a dispensing position, said dosing slide including a dosing cavity positioned underneath said funnel outlet of said medicament reservoir when said dosing slide is in said receiving position, said dosing cavity being movable into said dispensing position upon moving said protective cap from said closed position to said open position, said dosing cavity moving into said closure part and being surrounded by said closure part upon movement of said dosing slide into said dispensing position; a movable valve shield positioned inside said housing, said valve shield being movable between a rest position and a forward position in response to suction generated during inhalation, said valve shield moving said shutter when said valve shield moves into said forward position, the movement of said shutter releasing said dosing cavity from said closure part to thereby permit the release of dry powder from said dosing cavity into said channel of said mouthpiece and to permit the inhalation of dry powder by a user of said inhaler; locking means for locking said valve shield in said forward position only upon a defined minimum intensity of inhalation; return means for returning said dosing slide to said receiving position only after a correctly completed inhalation, thereby returning said dosing cavity underneath said funnel outlet; and a recording unit positioned inside said housing, said recording unit recording the number of correctly performed inhalations and having a protrusion that moves from an initial position to a final position such that, when said protrusion reaches its final position, the protrusion obstructs the return means from moving said dosing slide into its receiving position, thereby preventing further use of the inhaler; characterised in that said upper part and said lower part of the housing are joined together in a substantially airtight fashion by means of a mechanical fixing, such that a gas flow rate of no greater than 45 litres/minute, preferably no greater than 40 litres/minute, is required in order to propel a dose of said active agent from the reservoir to the mouthpiece for administration to the patient.

Suitably, the inhaler according to the second aspect of the present invention may be constructed such that a gas flow rate in the range of about 30 to about 40 litres/minute is required in order to propel a dose of said active agent from the reservoir to the mouthpiece for administration to the patient

According to a third aspect of the present invention, there is provided an inhaler for multiple dosed administration of a pharmacological dry powder, comprising a housing including an upper part and a lower part, a medicament reservoir containing the dry powder in loose form or in pre-dosed units for dispensing, a mouthpiece covered by a removable protective cap, and a movable dosing slide with a dosing cavity which can be positioned for filling in a starting position underneath a funnel outlet of the medicament reservoir; the arrangement being such that on partial opening of the protective cap, said powder is dosed into the dosing cavity; and on continued opening of the protective cap, the filled dosing cavity is transferred into a channel out of which a patient may inhale said dose of medicament; wherein a movable shutter and a movable valve shield are provided in the housing, which shutter is arranged to close the dosing cavity upon transfer of the dosing cavity into said channel; said valve shield being movable from its resting position on application of suction generated by inhalation, the arrangement being such that on application of a defined minimum level of suction, the valve shield is displaced and comes into contact with said shutter, whereby the shutter is caused to be displaced against adjustable locking means, thereby opening the dosing cavity such that a dose of powder is released therefrom for inhalation by a patient; and wherein means are provided for returning the dosing slide to the filling position with the dosing cavity under the funnel outlet only after correct completion of an inhalation; and wherein an integrated mechanical and/or electronic recording unit is provided, by means of which at least the correctly performed inhalations are recorded; which recording unit is arranged to effect a blocking of the inhaler after a defined number of inhalation doses have been used up; characterised in that said upper part and said lower part of the housing are joined together in a substantially airtight fashion by means of a mechanical fixing, such that a gas flow rate of no more than 45 litres/minute, preferably no more than 40 litres/minute, is required in order to propel a dose of said active agent from the reservoir to the mouthpiece for administration to the patient.

Suitably, the inhaler according to the third aspect of the present invention may be constructed such that a gas flow rate in the range of about 30 to about 40 litres/minute is required in order to propel a dose of said active agent from the reservoir to the mouthpiece for administration to the patient

According to a fourth aspect of the present invention, there is provided an inhaler for dispensing a dose of an active agent to the respiratory tract of a patient, which inhaler comprises a housing defined by an upper part and a lower part and a mouthpiece which is attached or is adapted to be attached to said housing; wherein said housing includes storage means for storing an active agent and an airway for delivering said dose from the storage means to the mouthpiece in a stream of air or other gas for administration to a patient; characterised in that said upper part and said lower part of the housing are joined together by means of a mechanical fixing and are sealed together by a separate sealing portion which serves to maintain a substantially airtight connection between said upper part and said lower part of the housing.

According to a fifth aspect of the invention, there is provided an inhaler for multiple dosed administration of a pharmacological dry powder, comprising a housing including a lower part and an upper part; a mouthpiece attached to an end of said housing, said mouthpiece including a channel extending therethrough; a protective cap covering said mouthpiece, said protective cap being movable between a closed position and an open position; a medicament reservoir located inside said housing, said medicament reservoir containing dry powder and including a funnel outlet; a movable shutter positioned inside said housing, said shutter including a closure part which protrudes into said channel of said mouthpiece; a movable dosing slide located inside said housing, said dosing slide being movable between a receiving position and a dispensing position, said dosing slide including a dosing cavity positioned underneath said funnel outlet of said medicament reservoir when said dosing slide is in said receiving position, said dosing cavity being movable into said dispensing position upon moving said protective cap from said closed position to said open position, said dosing cavity moving into said closure part and being surrounded by said closure part upon movement of said dosing slide into said dispensing position; a movable valve shield positioned inside said housing, said valve shield being movable between a rest position and a forward position in response to suction generated during inhalation, said valve shield moving said shutter when said valve shield moves into said forward position, the movement of said shutter releasing said dosing cavity from said closure part to thereby permit the release of dry powder from said dosing cavity into said channel of said mouthpiece and to permit the inhalation of dry powder by a user of said inhaler; locking means for locking said valve shield in said forward position only upon a defined minimum intensity of inhalation; return means for returning said dosing slide to said receiving position only after a correctly completed inhalation, thereby returning said dosing cavity underneath said funnel outlet; and a recording unit positioned inside said housing, said recording unit recording the number of correctly performed inhalations and having a protrusion that moves from an initial position to a final position such that, when said protrusion reaches its final position, the protrusion obstructs the return means from moving said dosing slide into its receiving position, thereby preventing further use of the inhaler; characterised in that said upper part and said lower part of the housing are joined together by means of a mechanical fixing and are sealed together by a separate sealing portion which serves to maintain a substantially airtight connection between said upper part and said lower part of the housing.

According a sixth aspect of the present invention, there is provided an inhaler for multiple dosed administration of a pharmacological dry powder, comprising a housing including an upper part and a lower part, a medicament reservoir containing the dry powder in loose form or in pre-dosed units for dispensing, a mouthpiece covered by a removable protective cap, and a movable dosing slide with a dosing cavity which can be positioned for filling in a starting position underneath a funnel outlet of the medicament reservoir; the arrangement being such that on partial opening of the protective cap, said powder is dosed into the dosing cavity; and on continued opening of the protective cap, the filled dosing cavity is transferred into a channel out of which a patient may inhale said dose of medicament; wherein a movable shutter and a movable valve shield are provided in the housing, which shutter is arranged to close the dosing cavity upon transfer of the dosing cavity into said channel; said valve shield being movable from its resting position on application of suction generated by inhalation, the arrangement being such that on application of a defined minimum level of suction, the valve shield is displaced and comes into contact with said shutter, whereby the shutter is caused to be displaced against adjustable locking means, thereby opening the dosing cavity such that a dose of powder is released therefrom for inhalation by a patient; and wherein means are provided for returning the dosing slide to the filling position with the dosing cavity under the funnel outlet only after correct completion of an inhalation; and wherein an integrated mechanical and/or electronic recording unit is provided, by means of which at least the correctly performed inhalations are recorded; which recording unit is arranged to effect a blocking of the inhaler after a defined number of inhalation doses have been used up; characterised in that said upper part and said lower part of the housing are joined together by means of a mechanical fixing and are sealed together by a separate sealing portion which serves to maintain a substantially airtight connection between said upper part and said lower part of the housing.

Suitably, the sealing action of said sealing portion may be such that a gas flow rate of about 30 to about 40 litres/minute, preferably about 45 litres/minute, and more preferably about 40 litres/minute, is required in order to propel a dose of said active agent from the reservoir to the mouthpiece for administration to the patient

Said sealing portion may comprise a flange provided on one of the upper part and the lower part, which flange is adapted to fit sealingly into a corresponding slot provided on the other of the upper part and the lower part. Thus, the upper and lower parts of the housing will overlap with one another at the sealing portion, ensuring an effective and air-tight seal.

Preferably, said mechanical fixing may be releasable. Said mechanical fixing may preferably comprise at least one snap-fit fixing which comprises a male portion provided on one of the upper part and the lower part and a co-operating female portion provided on the other of the upper part and the lower part. Advantageously, said inhaler may comprise a plurality of snap-fit fixings spaced around said housing.

Preferably, the male portion of each snap-fit fixing may comprise two co-extending substantially parallel arms which are optionally biased away from one another, and the female portion may comprise a recess into which said arms are adapted to be inserted, the arrangement being such that friction created by engagement of the arms with the walls of said recess acts to retain the arms within the recess. The male portion and female portion may be shaped to lock with one another upon interengagement Thus, the male and female portions may respectively be shaped such that upon insertion of the male portion into the female portion, one or more shaped protrusions on one of the male and female portions engages with a corresponding latch or recess on the other of the male and female portions, thereby locking the male portion in engagement with the female portion.

An active agent utilised in an inhaler according to the present invention may be in solid or liquid form. Preferably, said active agent may be a dry powdered substance.

The present invention encompasses both "passive" devices, which comprise an inlet for drawing air into said airway when suction is applied to said mouthpiece, such as to draw a dose of said active agent from the reservoir to the mouthpiece for administration to the patient; and "active" devices, which comprise a source of pressurised air or other gas which is adapted on activation to blow a dose of said active agent from the reservoir to the mouthpiece for administration to the patient, as well as devices with both "passive" and "active" functions or modes. The characteristics of both types of device are well known in the art and will be familiar to the skilled person. A "passive" device of the type modified by the present invention is, for example, disclosed in WO 97/20589.

According to a seventh aspect of the present invention, there is provided a kit of parts for assembling an inhaler according to any aspect of the invention, said kit comprising a mouthpiece, an upper part of a housing and a lower part of a housing, wherein said upper and lower parts are adapted to be fixed together according to the invention.

Following is a description by way of example only and with reference to the accompanying drawings of embodiments of the present invention.
Figure 1A shows a side view of an inhaler according to the invention, in the closed state.
Figure 1B shows a rear view of the inhaler of Figure 1A.
Figure 1C shows a front view of the inhaler of Figure 1A.
Figure 1D shows a plan view of the inhaler of Figure 1A.
Figure 1E shows a perspective view of the inhaler of Figure 1A, lacking the cap 950 and mouthpiece 920.
Figure 1F shows the inhaler of Figure 1A with the protective cap pulled out.
Figure 1G shows the inhaler of Figure 1A with the protective cap swung down fully, ready for inhalation.

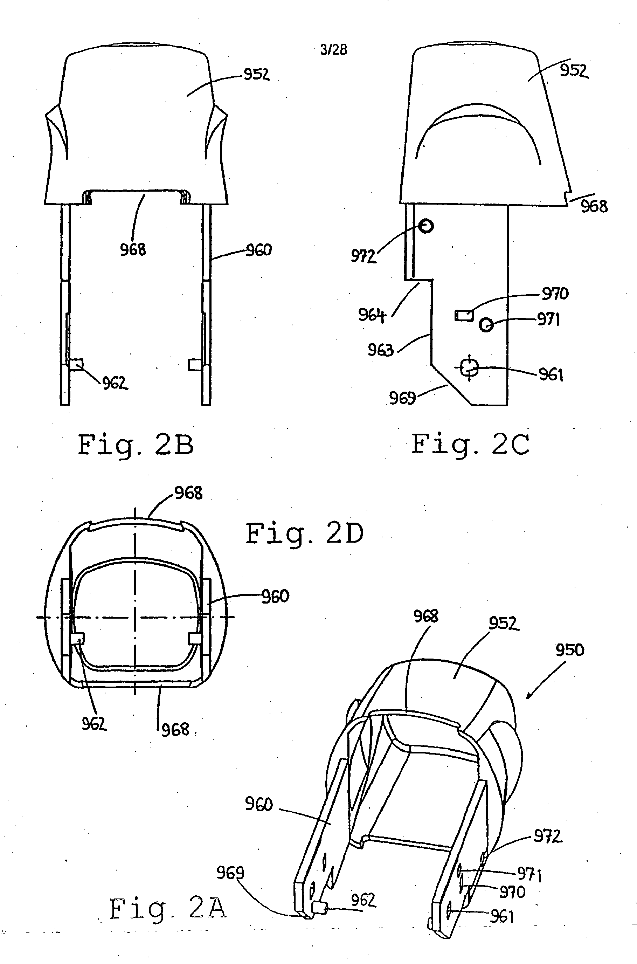

Figure 2A shows a perspective view of the protective cap of the inhaler of Figure 1A.
Figure 2B shows a plan view of the protective cap of the inhaler of Figure 1A.
Figure 2C shows a side view of the protective cap of the inhaler of Figure 1A.
Figure 2D shows a view into the protective cap of the inhaler of Figure 1A.
Figure 3A shows a perspective view of the lower part of the housing.
Figure 3B shows an enlarged view of one rear edge of the lower part of the housing.
Figure 4A shows a perspective view of the upper part of the housing.
Figure 4B shows an enlarged view of one rear edge of the upper part of the housing.
Figure 4C shows a side view, cut away, of the upper and lower parts of the housing in engagement with one another.
Figure 4D shows an enlarged view of the engagement between the upper and lower part of the housing.

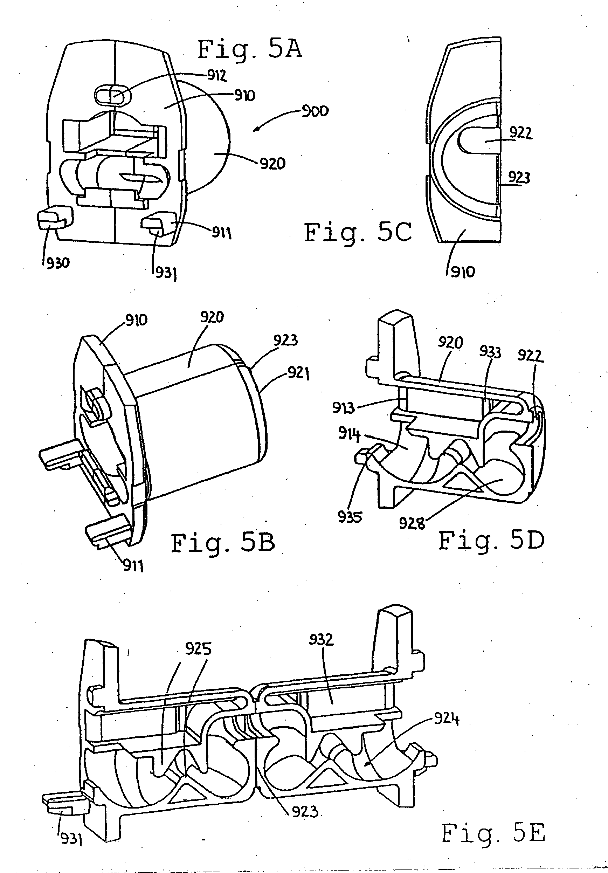

Figure 5A shows a perspective view of the base plate of the mouthpiece.
Figure 5B shows a perspective view of the side of the mouthpiece.
Figure 5C shows an external view of one half of the mouthpiece.
Figure 5D shows an internal view of one half of the mouthpiece.
Figure 5E shows an internal perspective view of the mouthpiece opened out.
Figure 6A shows a perspective view from below of the slide rail.
Figure 6B shows a perspective view from the side of the slide rail.
Figure 7A shows a perspective view from above of the carriage, shown laterally from the front.
Figure 7B shows a perspective view from above of the carriage, from the front.
Figure 7C shows a perspective view from above of the carriage, from the rear.
Figures 8A-8D show perspective views of part transverse cross-sections of a part of the inhaler of Figure 1A, showing the movement of the locking balls in response to movement of the carriage and rotation and tilting of the inhaler.

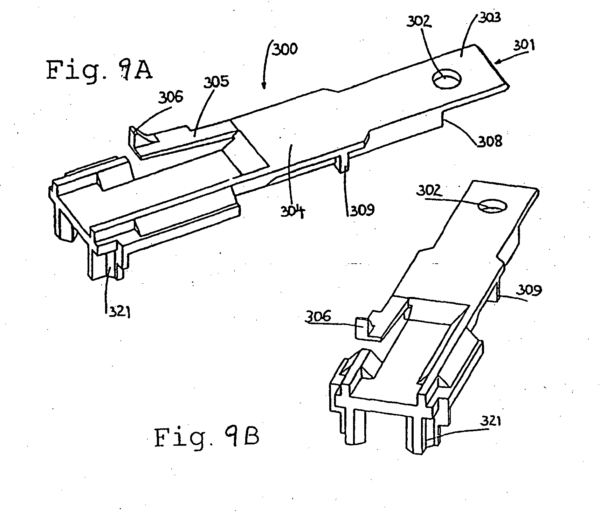

Figure 9A shows a perspective view from above of the dosing slide.
Figure 9B shows a perspective view from above of the dosing slide, shown from the rear.

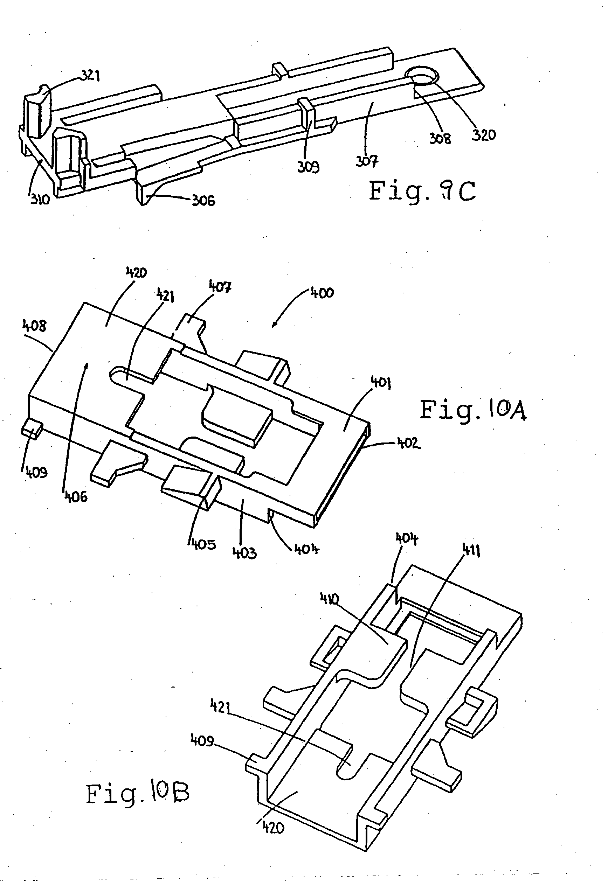

Figure 9C shows a perspective view from below of the dosing slide.
Figure 10A shows a perspective view from above of the shutter.
Figure 10B shows a perspective view from below of the shutter.

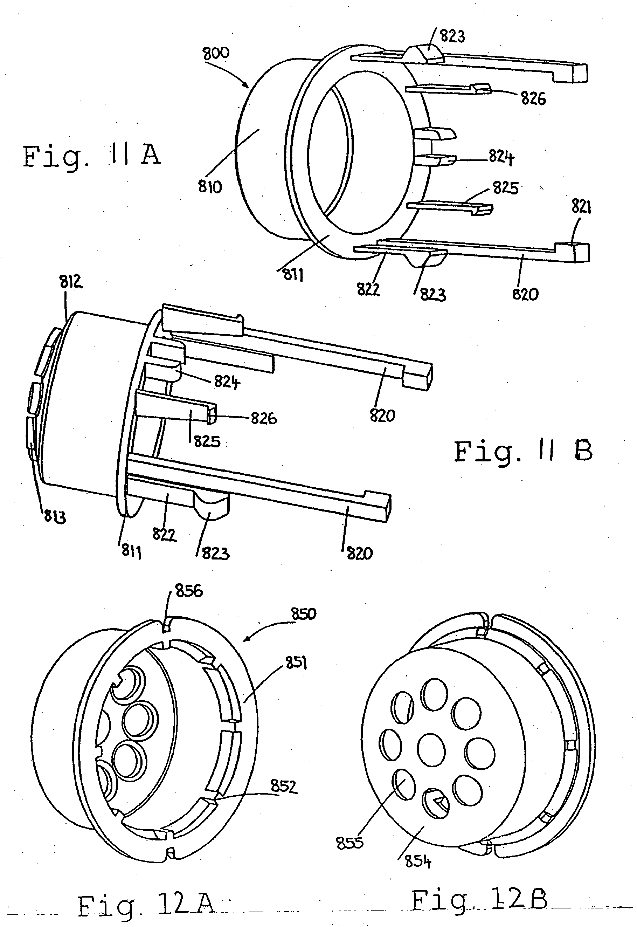

Figure 11A shows a perspective view of the valve shield.
Figure 11B shows a side perspective of the valve shield.
Figure 12A shows an inner perspective of the valve guide.
Figure 12B shows an outer perspective of the valve guide.
Figure 13A shows a perspective view from above of the funnel.
Figure 13B shows a perspective view from below of the funnel.
Figure 14A shows a perspective view from above of the funnel holder.
Figure 14B shows a side perspective of the funnel holder.
Figure 14C shows a perspective view from below of the funnel holder.
Figure 15A shows a perspective view from above of the funnel lid.

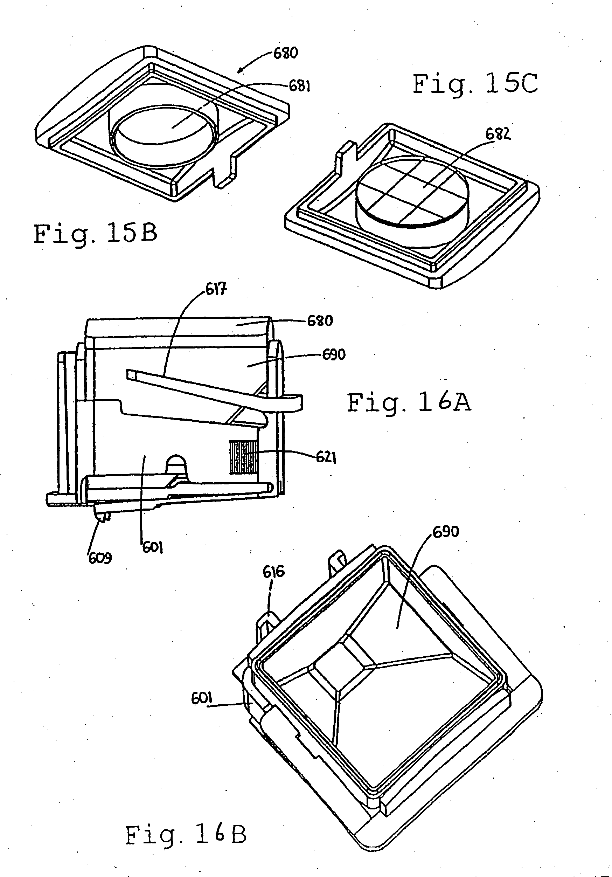

Figure 15B shows a perspective view from below of the funnel lid.
Figure 15C shows the funnel lid with semi-permeable membrane.
Figure 16A shows a side perspective view of the funnel holder, funnel and funnel lid.
Figure 16B shows a perspective view from above of the funnel holder and fitted funnel.

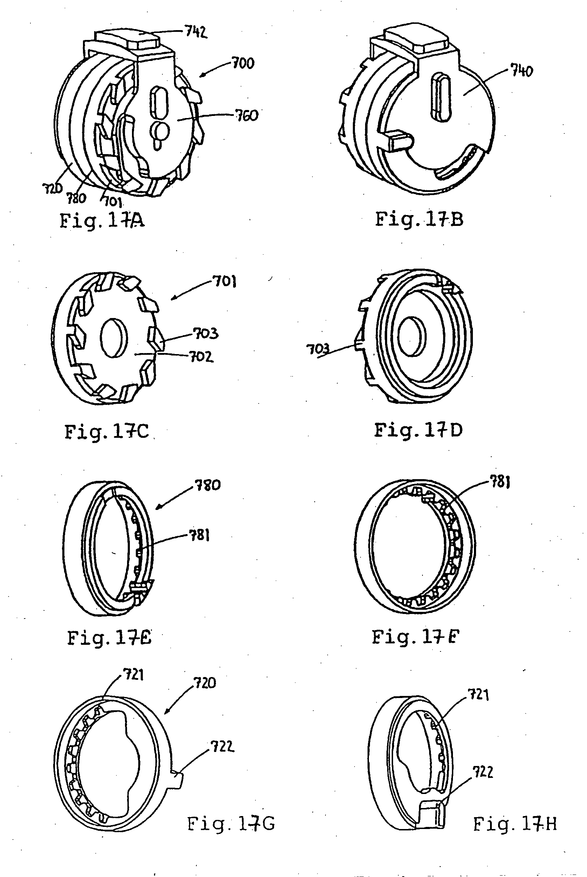

Figure 17A shows a perspective view of the units wheel of the counter.
Figure 17B shows a perspective view of the hundreds wheel of the counter.
Figure 17C shows an outer perspective view of the units wheel of the counter.
Figure 17D shows an inner perspective view of the units wheel of the counter.
Figure 17E shows an outer perspective view of the tens wheel of the counter.
Figure 17F shows an inner perspective view of the tens wheel of the counter.
Figure 17G shows an inner perspective view of the hundreds wheel of the counter.
Figure 17H shows an outer perspective view of the hundreds wheel of the counter.

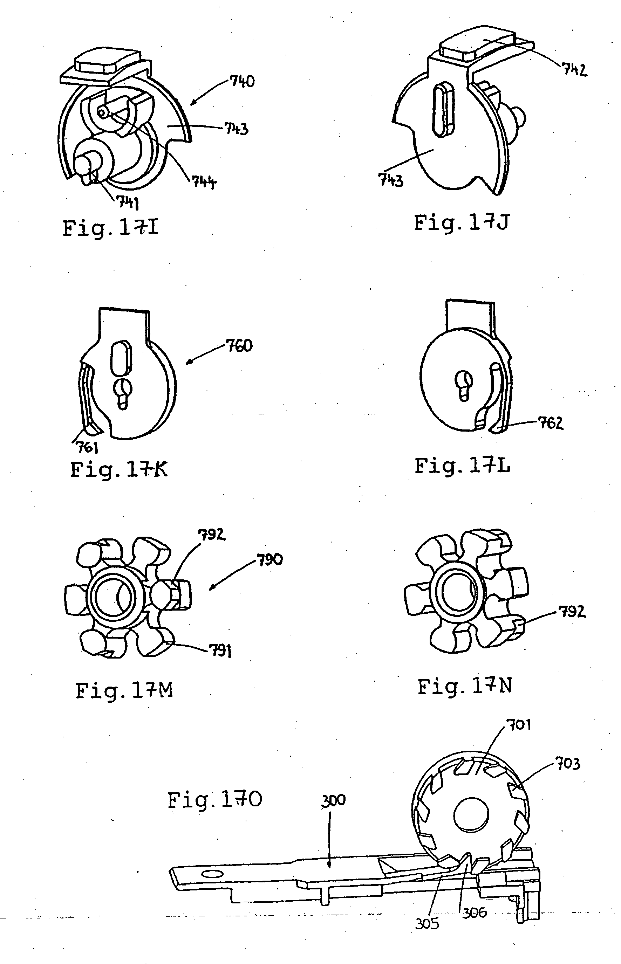

Figure 17I shows an inner perspective view of the counter body.
Figure 17J shows an outer perspective view of the counter body.
Figure 17K shows an outer perspective view of the cover plate of the counter.
Figure 17L shows an inner perspective view of the cover plate of the counter.
Figure 17M shows an outer perspective view of the drive wheel of the counter.
Figure 17N shows an inner perspective view of the drive wheel of the counter.
Figure 17O shows the engagement of the dosing slide on the counter, units wheel.
Figure 18A shows a horizontal longitudinal section view of the inhaler of Figure 1A along the line A-A in Figure 1A.
Figure 18B shows a vertical longitudinal section view of the inhaler of Figure 1A along the line B-B in Figure 1D.
Figure 18C shows a vertical transverse section view of the inhaler of Figure 1A along the line C-C in Figure 1D.
Figures 19A to 19D illustrate the functioning principle of the release of the shutter.

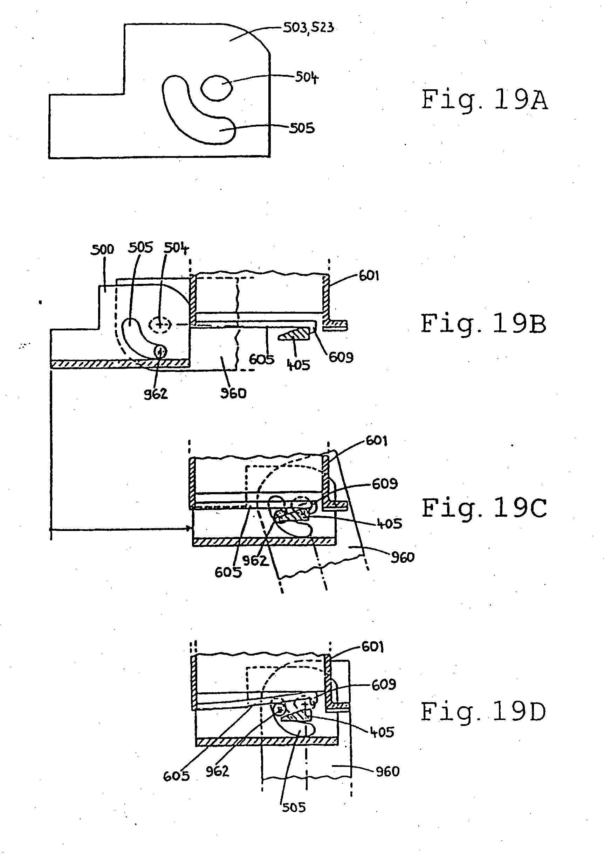

Figure 19A shows the side wings of the carriage with aperture and cam.

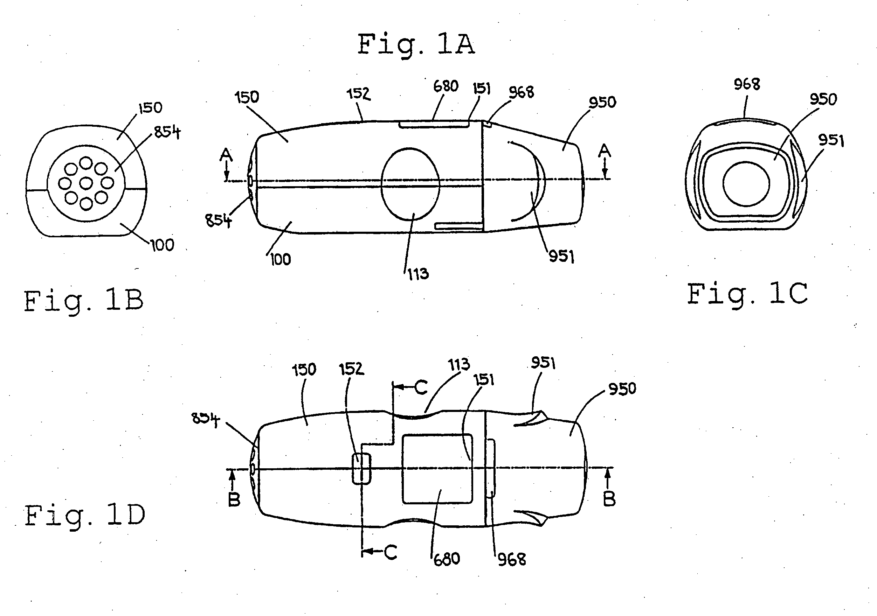

Figure 19B shows the inhaler of Figure 1A in closed configuration.
Figure 19C shows the shutter close to release, with the protective cap not swung down fully.

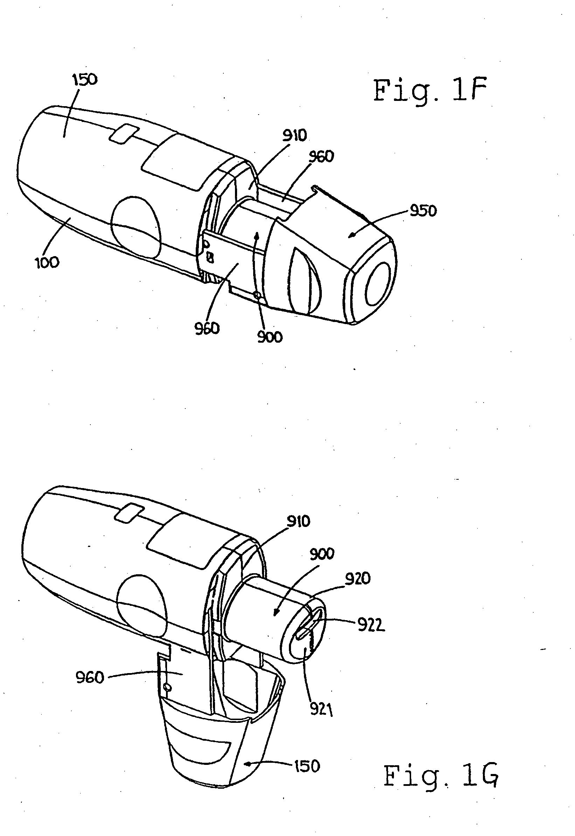

Figure 19D shows the shutter released, with protective cap swung down fully in accordance with Figure 1F.

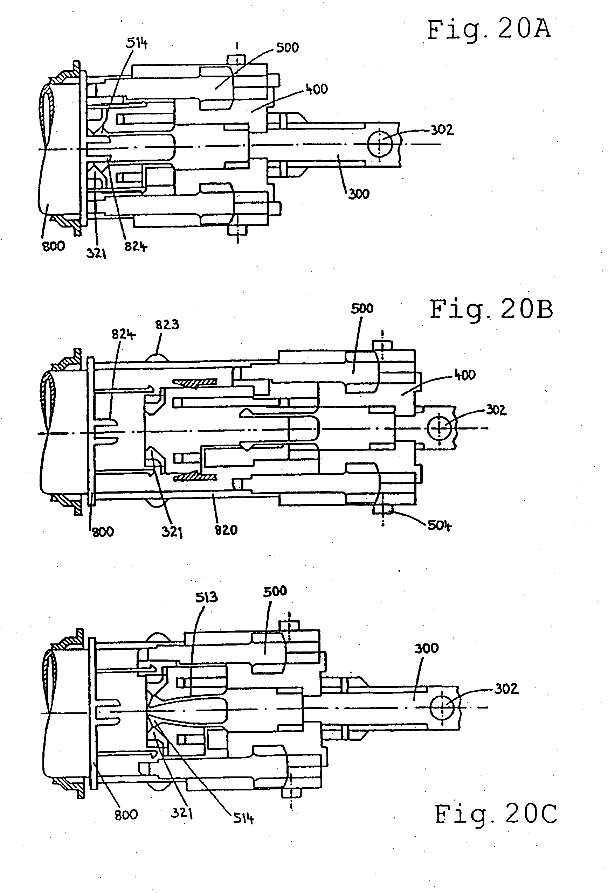

Figures 20A to 20F illustrate the functioning principle of the inhaler.

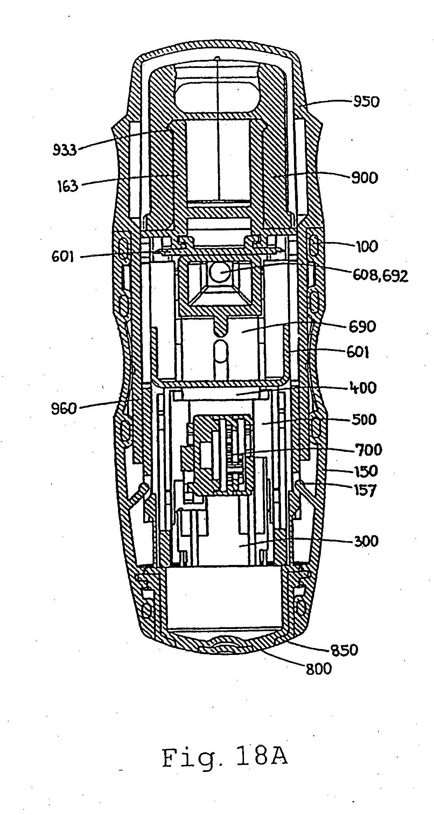

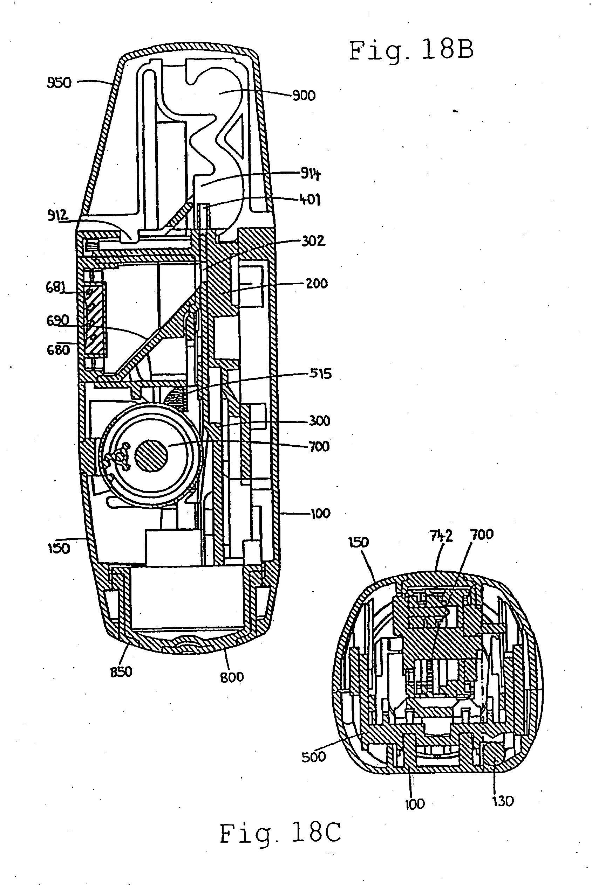

Figure 20A shows the inhaler closed in accordance with Figures 1A, 18A and 19B.
Figure 20B shows the inhaler open in accordance with Figures 1F and 19D.

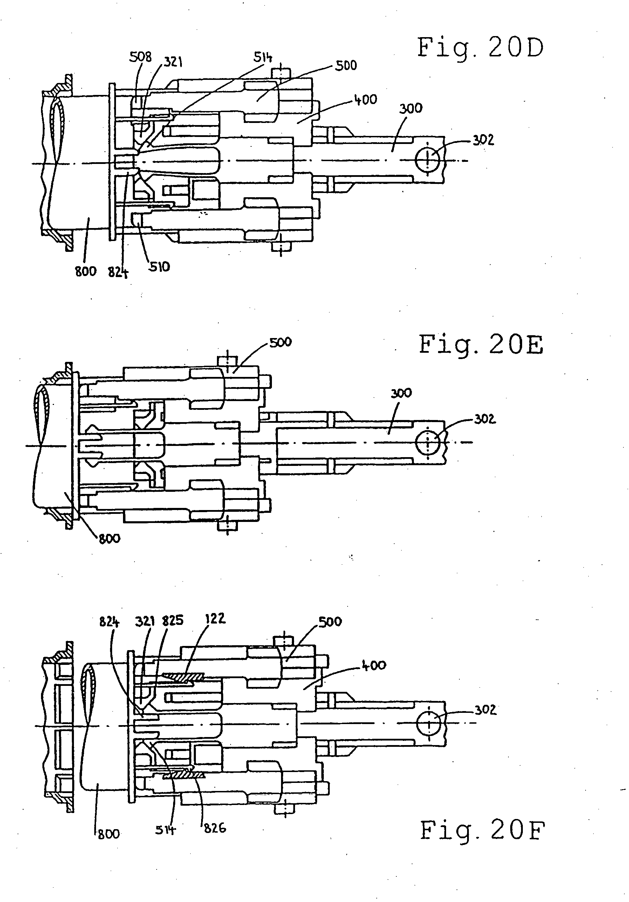

Figures 20C and 20D illustrate the closing of the inhaler after incomplete (20C) and complete (20D) inhalation.
Figures 20E and 20F show the inhaler closed after incomplete (20E) and completed (20F) inhalation.

### FIGS. 1A to 1B

Externally, the inhaler according to the invention is made up of the lower part 100 of the housing, the upper part 150 of the housing, and the protective cap 950. The lower part 100 of the housing and the upper part 150 of the housing have an elongate, semi-monocoque configuration. The upper part 150 has, on its top side, a fairly large opening 151 for receiving a funnel lid 680, and a window 152 through which the status of the counter can be read off. The lower part 100 of the housing and the upper part 150 of the housing are joined and sealed to one another in the manner described hereinbelow, such that a housing is obtained which is closed and substantially air-tight. Grip contours 951 are provided on the outside of the protective cap 950 to permit better gripping. Grip contours, preferably designed as grip dimples 113, are also arranged on both sides of the housing, in this case extending over the lower part 100 of the housing and the upper part 150 of the housing.

On the top of the protective cap 950, towards the outer edge, there is an elongate recess, by which means a clearance 968 is created together with the adjoining upper part 150 of the housing. By looking into this clearance 968 it is possible to ascertain if the mouthpiece is fitted and the clearance 968 is thus filled, or if the mouthpiece is missing and the clearance 968 is consequently open. Opposite the protective cap 950--on the rear part of the inhalerthe perforated base 854 of the valve guide enclosed by the lower part 100 of the housing and upper part 150 of the housing can be seen.

In the closed state shown here, the starting position--subsequently referred to as Situation A1--the protective cap 950 is fitted flush with the lower part 100 of the housing and the upper part 150 of the-housing. Thus, the medical preparation stored in the inhaler is protected quasi hermetically from external humidity.

### FIG. 1F

The inhaler has to be opened before use; to do this, the protective cap 950 is first of all pulled out in the axial direction. The line along which the protective cap 950 is pulled out is limited by a pair of side arms 960 which are fixed on the protective cap 950 and engage in a longitudinally displaceable manner in the inside of the inhaler. With the protective cap 950 pulled out this far, the mouthpiece 900 is already partly visible and is attached to the lower part 100 of the housing and the upper part 150 of the housing at the front and is enclosed on both sides by the side arms 960. As will be explained later, this step is associate with a temporary vibration for exact dosing of the medicament from the powder reservoir. This intermediate position, with the protective cap 950 pulled out, is hereinafter referred to as Situation A2.

### FIG. 1G

In order to allow the patient access to the mouthpiece 900, i.e. to permit inhalation, the protective cap 950 suspended on the side arms 960 has to be swung down in a further manoeuvre. The mouthpiece 900 with the mouth tube 920 protruding from the base plate 910 is now fully visible. The channel outlet 922 through which the patient inhales the medicament is situated on the end face 921 of the mouth tube 920.

In this position, with the protective cap pulled out and swung fully down--subsequently referred to as Situation A4--the inhaler itself is prepared for inhalation. The dose of medicament which has been made ready is in a loosened state. It should be understood that the protective cap 950 can only be swung down when it has first been pulled out to the limit. The dimensioning of the mouthpiece 900, the length of the side arms 960, and the sole possibility of swinging the protective cap 950 downwards, cause the patient by necessity to place the inhaler in the correct position. If the inhaler were used upside down in error, the patient would notice this immediately since his nose would hit against the protective cap 950 and he would thus barely be able to apply the mouthpiece 900.

Situation A3 characterizes the state in which the protective cap 950 is in its swing movement and has not yet reached its lowest position.

### FIGS. 2A to 2D

The protective cap 950 consists of the two aforementioned side arms 960 and the actual cap 952. The clearances 968, which provide space for the base plate 910 of the mouthpiece 900, are arranged on that edge of the cap 952 facing towards the mouthpiece 900, centrally on the top side and bottom side.

The two side arms 960 each extend laterally into the cap 952. At the front part, which engages in the inhaler, the side arms 960 have a special construction symmetrical to one another. Each side arm 960 has a rounded square aperture 961, a pin 962 lying below the rounded aperture 961 and directed inwards, a recess 963 incorporated from the underside of the side arm and having a cut edge 964, as well as forward bevels 969. Between the aperture 961 and the cut edge 964 in each side arm 960 there is a further rectangular aperture 970. Offset above this aperture 970 there is an outwardly directed dimple 971. The same type of dimple 972 is arranged in the lower area of the side arm 960 near the entry to the cap 952.

### FIGS. 3A to 3B

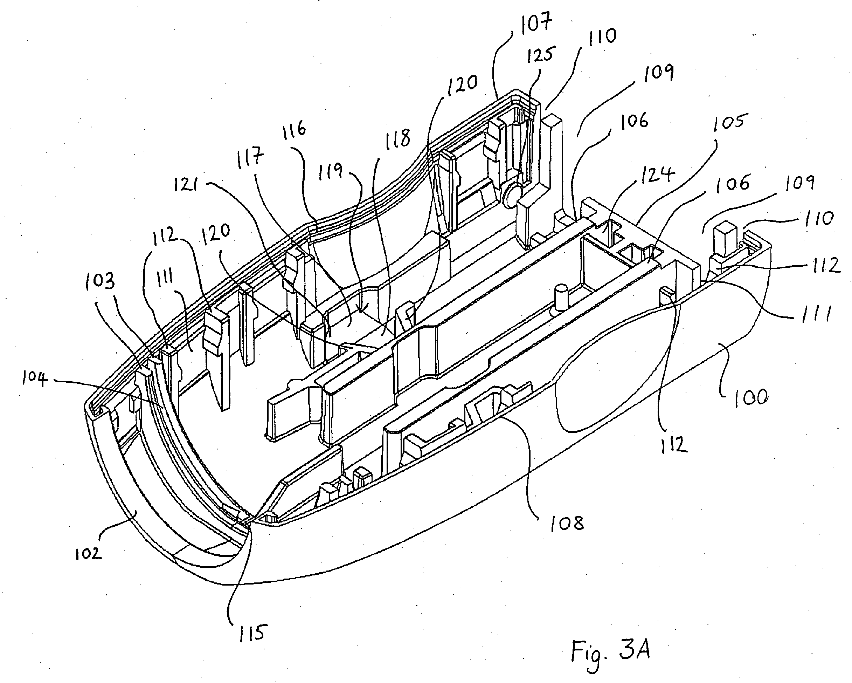

To the rear, the lower part 100 of the housing is strengthened at the end, as shown in Figure 3A, so that a semicircular bearing ring 102 is obtained. A double wall 103, likewise semicircular and with a radial receiving groove 104, is provided on the base at a distance from the bearing ring 102. Running centrally between the double wall 103 is a raised, axial connecting web 115.

Arranged on the base are two parallel bars or guide rails 106 which extend from the front side 105. The inner surface of the lower portion of the housing 100 contains a number of moulded features. In particular a housing profile 119 is moulded or pressed into the housing. This profile consists of an essentially flat surface 118 and a ramp 117 that extends upwards and outwards of the flat surface in the direction of the housing wall. Two angled walls 120 extend outward of the guide rails 106 in the direction of the housing wall but do not extend completely to the housing wall. A channel 121 is defined by the ends of the angled walls and the opposing housing wall. A blocking ball (not shown) sits in each of the flat surfaces 118 when the device is in an unlocked position and is retained in place by the walls 120 and the ramp 117.

At the front side 105, two receiving notches 109 are incorporated, as well as two axially extending longitudinal slots 110 in the base - near each housing wall. A safety cam 125 sits to the side at the entrance of each longitudinal slot 110. Between the two rails 106 and the front side 105 there are two U-shaped depressions 124.

Spaced along the inner face of each of the longitudinal sides 107, 108 of the lower part 100 of the housing are a plurality of recesses 111, each of which is defined by a pair of upstanding bars 112 which are provided with matching chamfered protrusions 114. The protrusions 114 face into the recess 111. An elongate groove 116 is provided extending longitudinally along the outer face of each of the longitudinal sides 107, 108 of the lower part 100 of the housing.

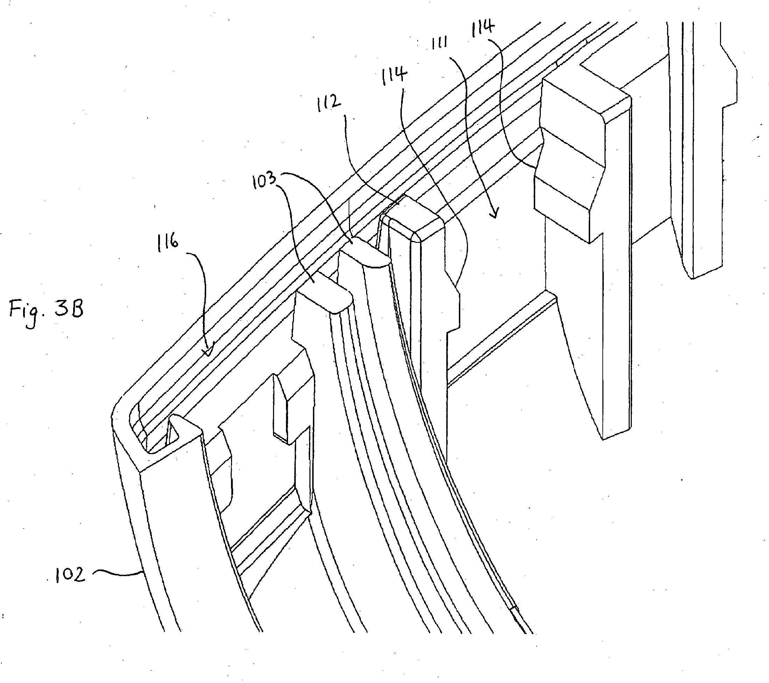

One edge of the rear end 102 of the lower part 100 of the housing is shown in Figure 3B. In this Figure, the shape and configuration of the recesses 111 and groove 116 as described above can be seen in more detail.

### FIGS. 4A to 4D

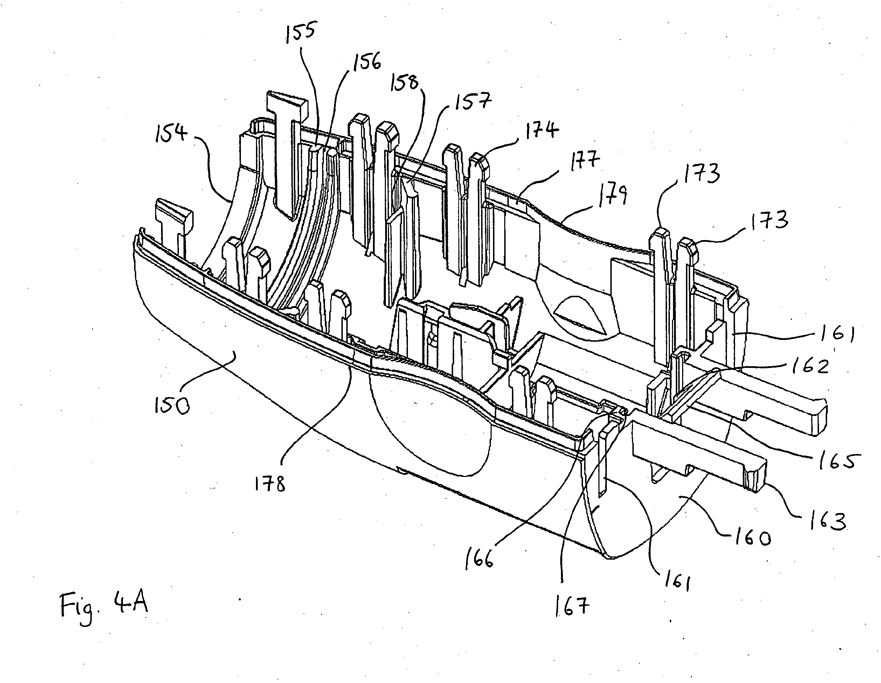

Analogously to the lower part 100 of the housing, the upper part 150 of the housing has a semicircular bearing ring 154 to the rear, as well as a double wall 155 with a receiving groove 156, as seen in Figure 4A. The half bearing rings 102 and 154, respectively, and the receiving grooves 104 and 156 combine to form full circles.

On the side walls, mounted ahead of the receiving groove 156, there are in each case a support cam 158 and a higher overspring rib 157. The support cam 158 and the overspring rib 157 project towards the centre of the upper part 150 of the housing and have a common point of origin on the side wall. Adjacent to the window 152 (not shown), two parallel supports 159 (not shown) spaced apart from one another are arranged on the interior of the upper part 150. In the interior of the upper part 150 there is also the recess 151 (not shown) for the funnel which is to be fitted. On both sides of this recess 151, towards the side walls, a limit cam 164 (not shown) in each case stands out from the interior of the upper part 150.

Corresponding to the longitudinal slots 110 in the lower part 100 of the housing, there are also two slots 161 in the front side 160 of the upper part 150 of the housing. In the front side 160 there is additionally a central receiving notch 162, and two elastic clamping prongs 163 extend from the front side 160 in the direction of the mouthpiece 900. A crosspiece 165 stretches between the front side 160 and the base of the clamping prongs 163, and adjacent to the receiving notch 162. To the rear of the front side 160, the clamping prongs 163 merge into a vertical U-profile 166, the vertical grooves 167 of the U-profiles 166 internally adjoining the front side 160 and facing one another.

Corresponding to the recesses 111 on the lower part 100 of the housing, the upper part 150 is provided with a plurality of pairs of arms 173, which are spaced along the longitudinal sides 177, 178 of the upper part 150 at intervals corresponding to the spacing of recesses 111 along the longitudinal sides 107, 108 of the lower part 100 of the housing. The two arms 173 of each pair are co-extending and substantially parallel to one another. Each arm is provided with a shaped head 174. An elongate flange 179, corresponding to the elongate groove 116, extends longitudinally along the outer face of each of the sides 177, 178 of the upper part 150 of the housing.

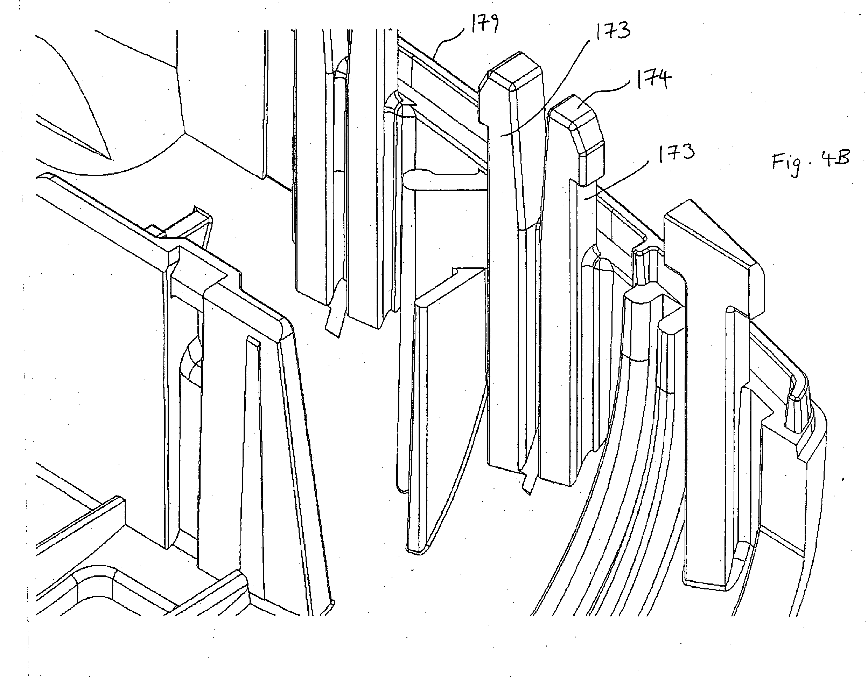

One edge of the rear end of the upper part 100 of the housing is shown in Figure 4B. In this Figure, the shape and configuration of the arms 173 and flange 179 as described above can be seen in more detail.

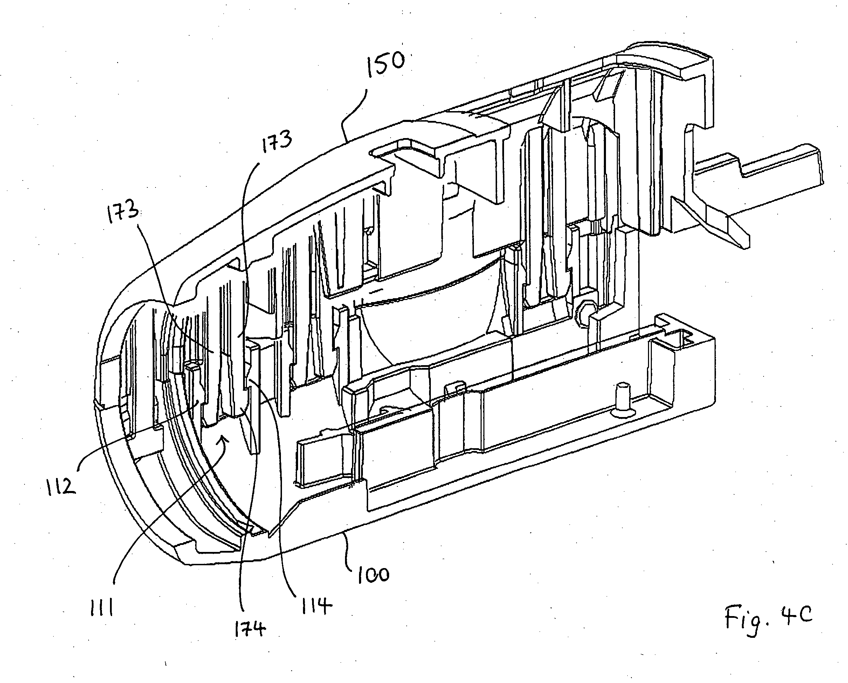

The arrangement is such that when the upper part 150 and lower part 100 of the housing are brought together, each pair of arms 173 on the upper part can be inserted into a corresponding recess 111 on the lower part, as illustrated in Figure 4C. The width of each recess 111 is such that the two arms 173 of each pair are pressed towards one another on insertion into the recess. This increases the friction between the arms 173 and the walls of the recess 111, resulting from the slight outward bias of the arms 173. The head 174 of each arm latches behind the chamfered protrusions 114, thereby securely locking the arms 173 within the recess 111. This serves to hold the upper part 150 and lower part 100 of the housing securely together. Meanwhile, each of the two elongate flanges 179 on the upper part 150 of the housing fits snugly into the corresponding elongate groove 116 on the lower part 100 of the housing, thereby creating an effective substantially air-tight seal between the upper and lower parts of the housing.

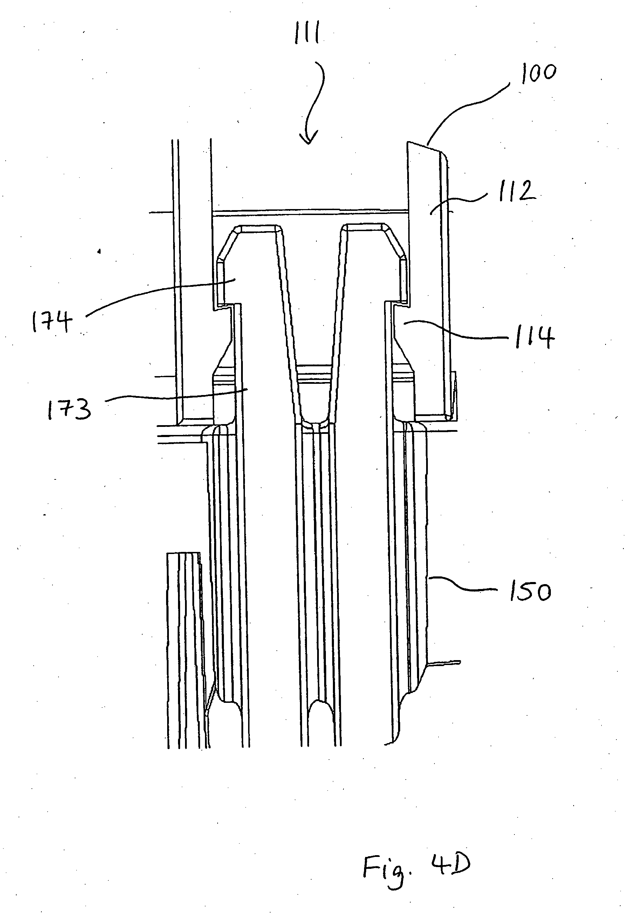

The engagement between the recesses 111 and arms 173 is shown in more detail in the enlarged view presented in Figure 4D.

### FIGS. 5A to 5E

The mouthpiece 900 consisting of the base plate 910 and the mouth tube 920 is advantageously made of two halves which are joined together, for example, by an integral film hinge 923 provided on the end face 921. On the base plate 910, facing the inhaler, there is a full connector plug 911 at the bottom of each half, and a half-cam 912 at the top, which complements the adjacent half-cam 912. Each connector plug 911 has at its front free end, in the lower area, a recess 930 with an inwardly directed bevel 931.

Incorporated underneath the two half-cams 912 is an engagement opening 913 which extends as a shaft 932 right into the mouth tube 920. Deeper in the shaft 932, a recessed groove 933 is present in each case laterally in the wall of the mouth tube 920. The channel inlet 914 for the atomizer path 924 of the mouth tube 920 lies below the engagement opening 913. The channel inlet 914 is connected to the channel outlet 922 via the atomizer path 924. A horizontal ramp 935, complementing the second half of the mouthpiece, is in each case arranged on the base plate 910 under the channel inlet 914.

Inside the atomizer path 924, behind the channel inlet 914, there are a plurality of baffles 925 which protrude into the atomizer path 924 for impacting the medicament-containing air stream and causing it to swirl, so that the atomizer path 924 acquires a course which is rich in curves. Nearer the channel outlet 922, the powder aerosol flowing through enters via an S-curve into an enlarged channel section 928 and is here deflected to the channel outlet 922. The purpose of the special configuration of the atomizer path is to deagglomerate the powder and to reduce the flow rate of the powder so as to deposit coarser particles which are ineffective for inhalation. At the same time, the impacting of particles of active substance in the pharynx of the patient is thus prevented. The mouth tube 920 is flattened off horizontally, at least in the area of the end face 921, so that the patient is induced to employ the correct positioning of the inhaler during use.

### FIGS. 6A and 6B

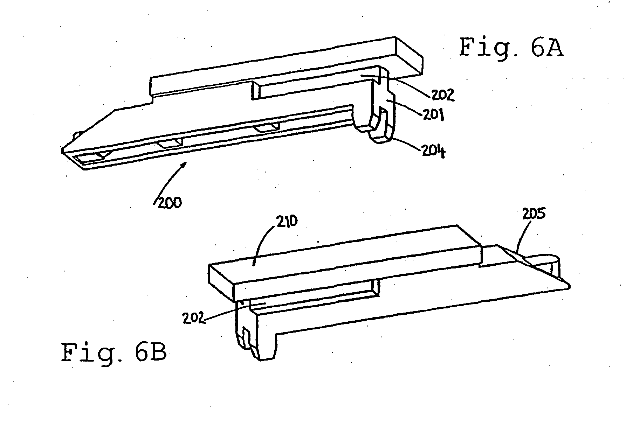

The slide rain 200, shown in Figures 6A and 6B, has two longitudinal grooves 202 which extend laterally from the end face 201 and parallel to one another, and which reach to approximately halfway along the slide rail 200. In a continuation of the end face 201, two feet 204 spaced apart from one another extend downwards from the bottom of the slide rail 200. At the top, the slide rail 200 has a ledge-like roof part 210 which protrudes over the end face 201 in the matter of a projecting roof. At the opposite end from the end face 201, the slide rail 200 ends in a sloping surface 205 which merges ramp-like with the top surface of the slide rail 200, the roof part 210 ending in front of the sloping surface 205.

### FIGS. 7A to 7D

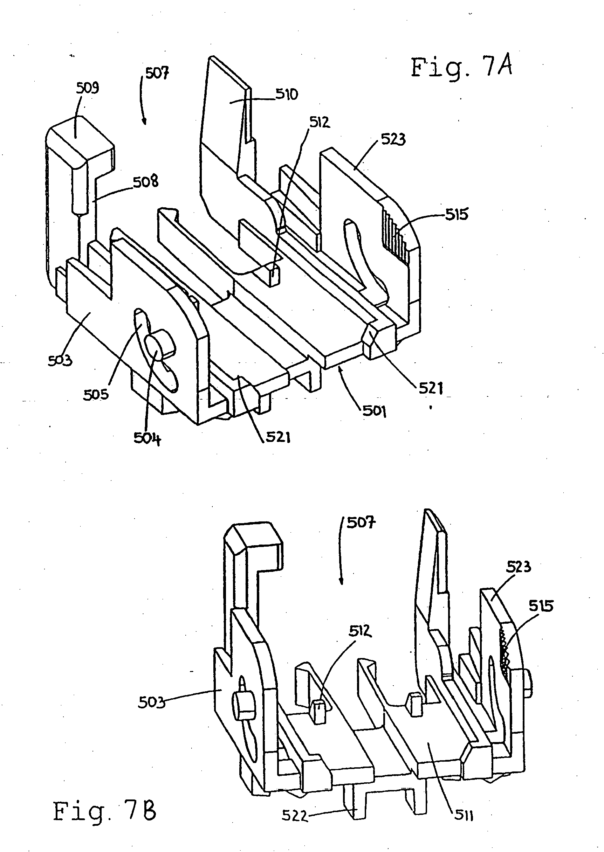

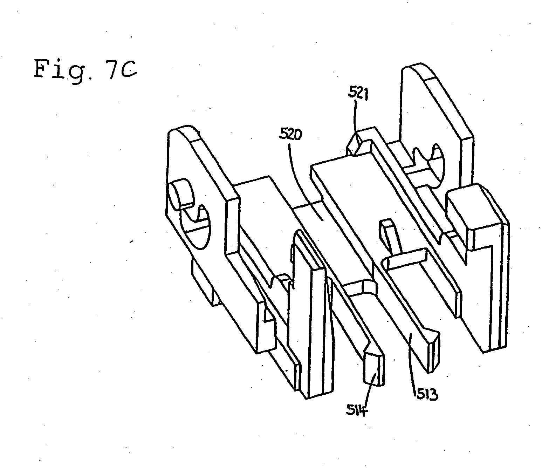

The carriage 500, shown in Figures 7A to 7D, has two opposing side walls or wings 503, 523 rising laterally and beginning on the front side 501, with an outwardly directed cam 504 being in each case arranged thereon. The cam 504 is wider in the horizontal than in the vertical, so that an approximately oval shape is obtained. An arcuate slot or aperture 505 is incorporated in each of the wings 503, 523 and runs round the cam 504 from below in a part circle. The cams 504 are arranged to engage with slots provided in the side arms 960 of the cap 950, whilst the slots 505 are arranged to engage with projections provided on the side arms 960; thereby engaging the carriage with the cap. Through such co-operation between the slots and projections, the movement of the cap as it is pulled open is communicated to the carriage, which is in this way able to move laterally in conformity with the cap. Once the cap has been extended fully, it is capable of being swung through 90 degrees as shown in Figure 1G to permit user access to the mouthpiece.

At its rear side 507, the carriage 500 has two laterally rising struts 508, 510, the strut 508 having a horizontal shoulder 509. Two pull cams 512 rise from the carriage base 511, while to the rear side 507 the carriage base 511 continues in the form of two elastic spring tongues 513 which end as spring wedges 514 and can be deflected out to the sides. Between the two spring tongues 513, a longitudinal groove 520 extends centrally through the carriage base 511. A pull catch 521 is present on the front side 501 between the groove 520 and each of the wings 503, 523. Towards the front side 501, the wing 523 has a grate section 515 on the inside.

Underneath the groove 520, the carriage 500 has two runners 522 on its underside, which runners 522 engage guide rails 106 provided in the lower part 100 of the housing, such that the carriage can run in a lateral direction along the rails. The carriage also comprises abutment portions 524, which provide the first locking element of the gravity-actuated locking mechanism more fully described below.

### FIGS 8A to 8D

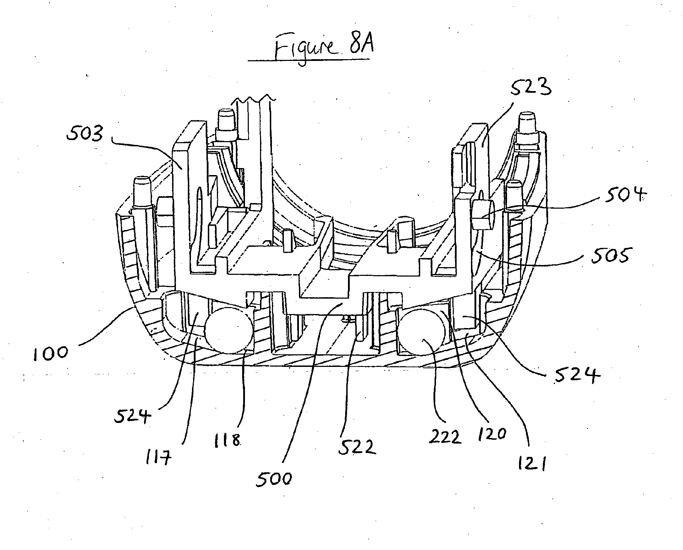

In Figure 8A, the carriage 500 is shown (not in section) mounted in the lower housing portion 100 on the runners 522. In such a mounted position the abutment portions 524 extend downwards towards the base of the lower part of the housing 100 and are adapted to pass through the channels 121 when the carriage is moved laterally back and forth along the guide rails in conformity with the movement of the cap 950. As hereinbefore described, movement of the cap 950 is communicated to the carriage by the side arms 960 which are connected to the carriage by means of the cams 504 and the slots 505. For clarity, the arms 960 of the cap are not shown connected in Figure 8A. In this Figure, the carriage is in a fully retracted position representing the situation when the cap is closed. In this arrangement the abutment portions 524 do not extend into the channels 121. Locking balls 222 form a second locking element of the gravity-actuated locking mechanism and are shown in this figure in their unlocked positions such that if the cap was open causing the carriage to move, the abutment portions would move through the channel unimpeded.

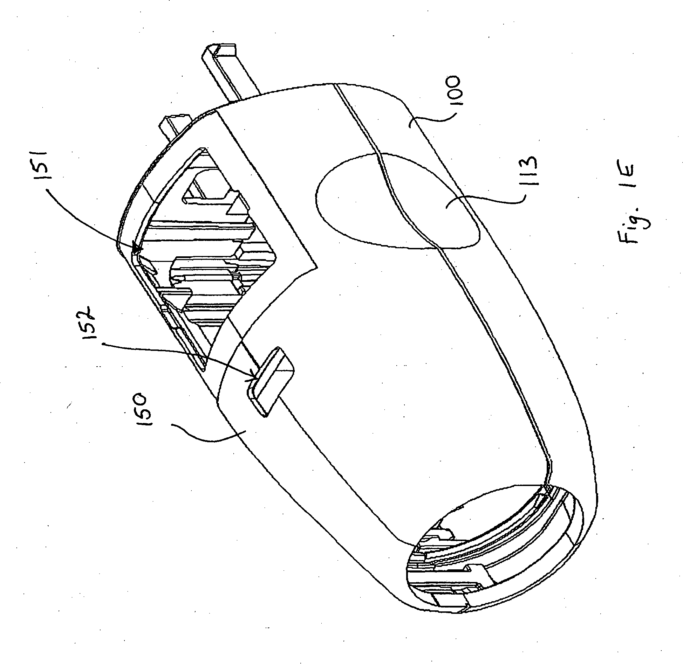

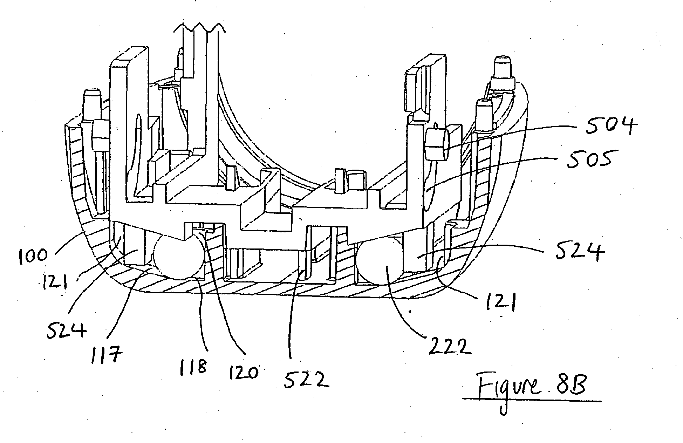

Figure 8B is substantially the same view as that shown in Figure 8A, except that the carriage 500 is drawn forward as a result of the cap 950 being drawn laterally outwards away from the housing to assume the position shown in Figure 1E. In Figure 8B, the abutment portions 524 can be seen as having advanced with the carriage through the channels 121, past the blocking balls 222. Again, in this Figure the blocking balls are in the unlocked position and the abutment portions 524 move past the balls unimpeded allowing the cap to open.

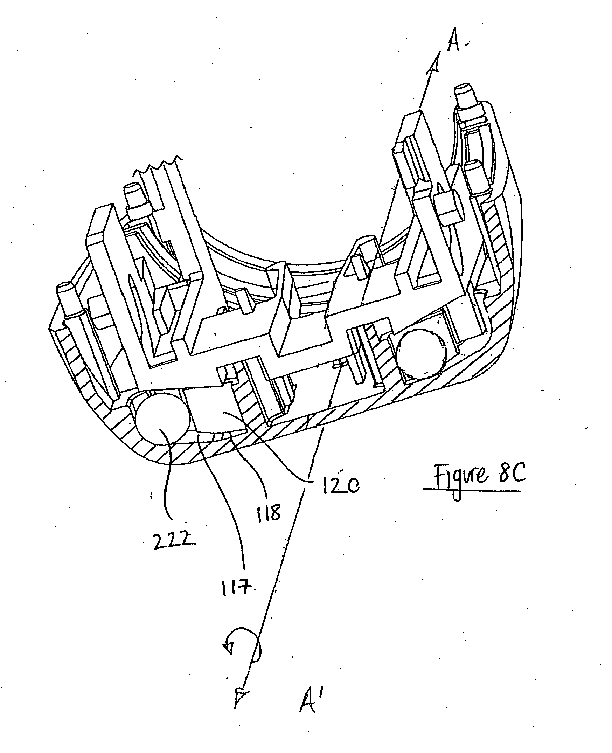

Figure 8C shows a view substantially the same as that shown in Figures 8A and 8B.. The device has been rotated about the axis A-A' in an anti-clockwise direction. The blocking ball 222 shown on the left of the Figure can be seen to move off the flat surface 118 of the lower portion 100 of the housing and across the ramp 117 in the direction of the wall of the housing portion 100. The rotation must be sufficient for the blocking ball 222 to overcome the inertia of moving across the ramp 117. After reaching the critical point of rotation the ball moves under gravity into the position shown. Naturally, the skilled person will appreciate that the sensitivity of the locking mechanism can be adjusted by increasing or decreasing the inertial forces by either altering the mass of the blocking ball 222, or by altering the slope of the ramp 117, altering the contact angle between ball and housing profile or any combination of these. This movement occurs as a result of the rotation of the device. With the blocking ball 222 located on the ramp 117, any attempt to move the cap 950 and therefore the carriage 500 is impeded because the abutment portion 524 would come into contact with the blocking ball 222. In this manner, if a user has rotated the device out of the appropriate orientation for correct dosing the cap cannot be removed. It follows from the above that if the device is tilted in the clockwise direction, locking is effected by movement of the blocking ball 222 located on the right of the Figure into the path of the opposed abutment portion.

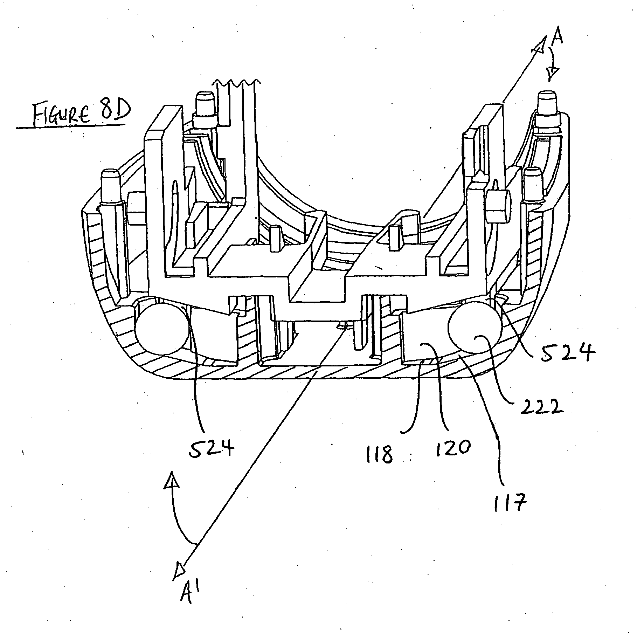

Figure 8D shows a view substantially the same as that of Figures 8A, 8B and 8C. In this Figure however, the device has been tilted (as opposed to being rotated) out of the axis A-A' as if the cap end of the device was tilted upwards. In response to this tilting movement the blocking balls 222 both move across the ramp 117 guided by the angled walls 120. Once again, with the blocking balls 222 positioned on the ramp 117 in the channels 121, any attempt to move the cap 950 will result in the abutment portions 524 contacting the blocking balls 222 and actuate the locking mechanism. The sensitivity of the locking mechanism to the tilting motion of the device can be adjusted in the same manner as was discussed above in the case of the rotating motion.

### FIGS. 9A to 9C

Near its front edge 301, the tongue-like dosing slide 300 has a through-bore, the dosing cavity 302. Startling from the front edge 301, the dosing slide 300 extends firstly as a narrow tongue tip 303 and then widens to the rear part 304. A spring leaf 305, from which a cam 306 rises perpendicularly, is arranged on the outside flank of the rear part 304.

On the underside, the dosing slide 300 has flank ridges 307 which begin immediately behind the dosing cavity 302 and there form limit stop edges 308. The dosing cavity 302 is surrounded by a radial sealing rim 320 on the underside. Near the transition to the rear part 304, there are two downwardly extending transverse cams 309. On the rear edge 310 of the dosing slide 300 there are two downwardly directed, profiled catches 321.

### FIGS. 10A and 10B "

The shutter 400 has the function of releasing the medicament made ready in the dosing cavity 302 only when there is a suitably forceful inhalation. At the very front, the shutter 400 has a sleeve-like closure part 401 with the through-opening 402. Behind the closure part 401 there are outer, vertical side ridges 403, beginning with a limit stop 404. An outwardly directed and ramp-shaped wing 405 is arranged on each side ridge 403. In the direction of the rear part 406, the wing 405 is followed by a side bracket 407. At the bottom, on the rear edge 408, the shutter 400 also has an outwardly directed carrier 409. On the bottom, in the area of the wings 405, two base plates 410 extend towards one another, leaving a through-gap 411. Towards the rear edge 408, the end of the rear part 406 is provided with a cover 420 which at the top stretches across the two parallel side ridges 403 and begins approximately in the area of the side brackets 407. In the cover 420 there is a rounded-off groove 421 which extends along the middle and which is open to the centre of the shutter 400.

### FIGS. 11A and 11B

The valve shield 800 has the function of inducing the patient to generate a defined minimum suction for a correct inhalation. The valve shield 800 consists of a cylindrical capsule 810 and a plurality of arms attached thereto and having different tasks. The capsule 810 has a flange-like collar 811 surrounding the opening and protruding outwards. The bottom 812 of the capsule 810 is convexly curved outwards and has a large number of raised stubs 813 on the outside.

The further elements of the valve shield 800 are attached perpendicularly onto the collar 811 and extend in the axial direction. On the collar 811 there is firstly a pair of long tentacles 820 which lie opposite one another and which have carriers 821 at their very front. Before the tentacles 820 there are two shorter spring arms 822 with outwardly directed wedge profiles 823 at their tips. Behind the tentacles 820 there are two short locking teeth 824 standing close to one another.

### FIGS. 12A and 12B

The capsule-shaped valve guide 850, when fitted into the lower part 100 of the housing and the upper part 150 of the housing, serves to receive the valve shield 800, i.e. its capsule 810. To this extent the valve guide 850 has the function of a slide bearing. Complementing the collar 811 of the valve shield 800, the valve guide 850 has an external limit stop flange 851. Slide ribs 852 in the inside of the valve guide 850 have the purpose of reducing the friction as the valve shield 800 travels out. The perforated bottom 854 has numerous holes 855, so that the stubs 813 of the valve shield 800 find space therein. At the top and bottom of the limit stop flange 851 there are two diametrically opposite notches 856. The holes 855 and the stubs 813 make it possible to design the inhaler virtually closed at the rear and thus to prevent the penetration of dirt particles and the inadvertent displacement of the valve shield 800.

### FIGS. 13A and 13B

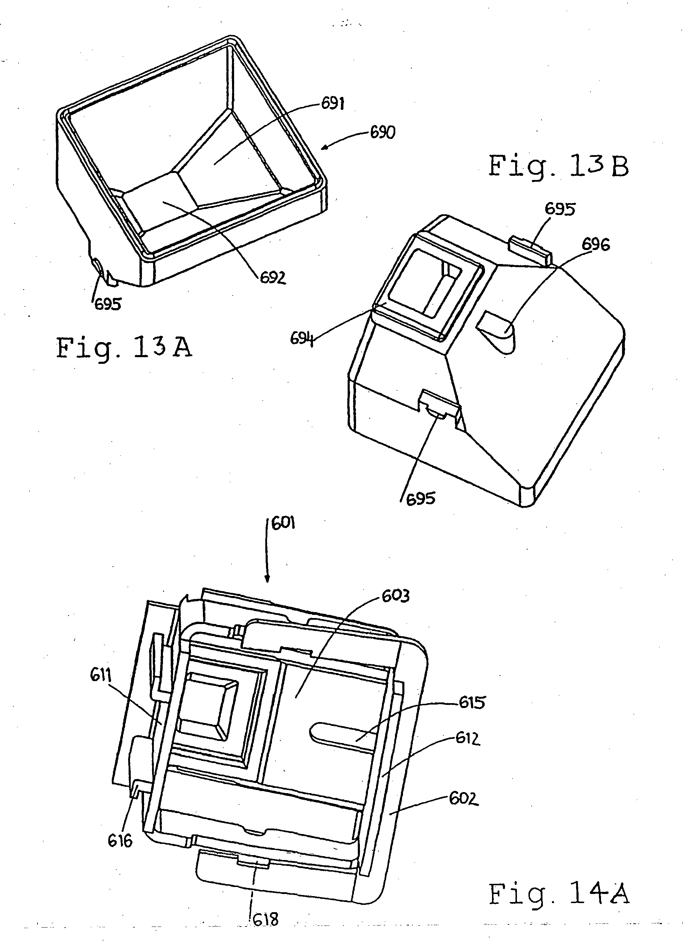

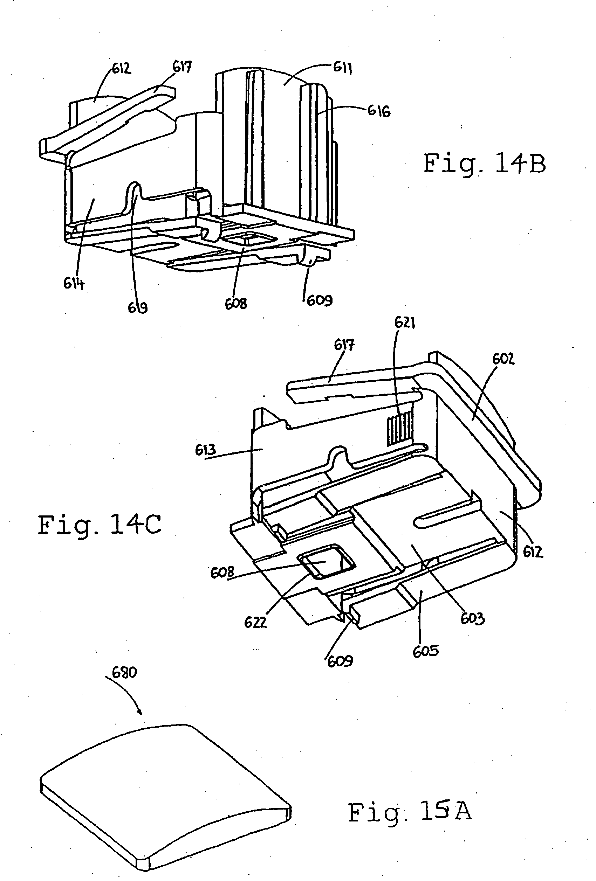

The funnel 690 is intended for fitting into the funnel holder 601 (see FIGS. 14A, 14B). The funnel bottom 691 is designed sloping obliquely towards the outlet 692, so that the medicament powder flows in a favourable manner. The outlet 692 is surrounded on the outside by a sealing element 694. On the outside, the funnel 690 has retainer cams 695 projecting upwards on two opposite sides, as well as a centrally positioned fixing nose 696 which sits on the oblique funnel bottom between the retainer cams 695.

### FIGS. 14A to 14C

The box-shaped funnel holder 601 has, on the underside, the holder bottom 603, the front wall 611, the rear wall 612, and the two half-height side walls 613, 614 lying between the front and rear walls 611, 612. Located in the holder bottom 603 are the funnel outlet 608 and an elongate groove 615. At the very bottom, a sealing member 622 surrounds the funnel outlet 608, so that it is possible to prevent the entry of humidity and the escape of powder from the funnel 690 onto the sliding surfaces of the dosing slide 300.

Arranged perpendicularly on the front wall 611 are two angled rails 616, and at the top of the rear wall 612 there is a support edge 602, from which a spring arm 617 in each case extends along the two side walls 613, 614. A groove 618 facing the respective side wall 613, 614 is provided in each of the spring arms 617. The side walls 613, 614 each have a downwardly open notch 619 approximately at their centre on the lower edge. A grate section 621 is provided on one side wall 613 near the rear wall 612. Two elastic lamellae 605 are connected to the rear wall 612 and extend along the outer flanks of the holder bottom 603. The lamellae 605 have vertically movable ends with downwardly projecting blocking cams. 609.

### FIGS. 15A to 15C

The funnel lid 680 serves to close the funnel 690. On the underside of the funnel lid 680 there is a chamber 681 which is closed by a semi-permeable membrane 682. The chamber 681 is intended to receive a moisture-attracting desiccant powder and the moisture can diffuse through the semi-permeable membrane 682.

### FIGS. 16A and 16B

When the funnel arrangement is in its completed state, the funnel 690 is fitted into the funnel holder 601, and the funnel 690 is closed by the funnel lid 680. However, the sequence of assembly of the inhaler need not include the prior completion of the funnel arrangement.

### FIGS. 17A and 17B

The complete counter 700 consists of the units wheel 701, the tens wheel 780, the hundreds wheel 720, the counter body 740, the counter cover plate 760, and two identical drive wheels, which are not shown here. The purpose of the counter 700 is to register the number of doses used or doses still available and to indicate to the patient the inhalation which has just taken place, as long as it was performed correctly. Numbers and, if appropriate, a colour marking are provided on the circumference of the counter wheels 701, 780 and 720 fixed on the axle 741 of the counter body 740. The current status of the counter is displayed under a lens 742 which sits in the window 152 of the upper part 150 of the housing. The lens 742 is connected to the counter body 740.

### FIGS. 17C and 17D

The units wheel 701 has ten radially distributed cams 703 on its outer surface 702.

### FIGS. 17E and 17F

The tens wheel 780 with its inner toothed ring 781 is itself of a conventional construction.

### FIGS. 17G and 17H

The hundreds wheel 720 likewise has an inner toothed ring 721 and an outwardly protruding end cam 722.

### FIGS. 17I and 17J

The counter body 740 consists of the base plate 743, the lens 742 set on the top at right angles, the axle 741 extending perpendicularly from the base plate 743, as well as the drive-wheel bearing 744.

### FIGS. 17K and 17L

Bearing on the units wheel 701, the counter cover plate 760 is fixed on the axle 741 of the counter body 740. The counter cover plate 760 has an elastic adjusting tongue 761 with a wedge cam 762 at the end, which cam 762 moves at all times between two cams 703 of the units wheel 701.

### FIGS. 17M and 17N

The star-shaped drive wheel 790 is fitted on the one hand between the units wheel 701 and the tens wheel 780 and on the other hand between the tens wheel 780 and the hundreds wheel 720. It has six uniformly arranged teeth 791, of which every second tooth 791 has an undercut 792 at its tip on one side of the drive wheel 790.

### FIG. 170

When a correct inhalation has been completed and the inhaler has been closed again by swinging the protective cap 950 upwards and pushing it in, the actuation of the counter 700 takes place. Only on pushing the dosing slide 300 back into the starting position--Situation A1--is a cam 703 on the units wheel 701 gripped by the cam 306 situation on the spring leaf 305, and the units wheel 701 thereby turned by one count position.

When the intended number of doses have been taken from the inhaler, the hundreds wheel 720 is in such a position that the end cam 722 has positioned itself at the far top and, on pulling the carriage 500 out, the shoulder 509 (see FIGS. 7A to 7B) strikes against the end cam 722. Further actuation of the inhaler is thus blocked.

### FIGS. 18A to 18C

In the assembled state, the following arrangement obtains in Situation A1. The lower part 100 of the housing and the upper part 150 of the housing are joined together as hereinbefore described. The mouthpiece 900 is inserted from the front and the protective cap 950 is fully closed.

The slide rail 200, the carriage 500, the dosing slide 300, the shutter 400 lie in the housing parts 100, 150. The valve guide 850 is fitted, and therein the valve shield 800, as well as the complete funnel arrangement--consisting of funnel 690, funnel holder 601 and funnel lid 680--and the counter 700. The valve shield 800 is in its rearmost position, and the dosing slide 300 is located such that the dosing cavity 302, positioned under the funnel outlet 608, can fill with medicament. The closure part 401 of the shutter 400 protrudes into the channel inlet 914. The clamping prongs 163--additionally fixing the mouthpiece 900-- sit in its shaft 932 and engage in the grooves 933. The half-cams 912 of the mouthpiece 900, joined together, are locked in the receiving notch 162 in the upper part 150 of the housing. The connector plugs 911 of the mouthpiece 900 pass through the receiving notches 109 in the lower part 100 of the housing, and the ramp 935 of the mouthpiece 900 engages under the front edge of the roof part 210 of the slide rail 200. The side arms 960 of the protective cap 950 protrude through the slots 110 in the lower part 100 of the housing and through the slots 161 in the upper part 150 of the housing and embrace the wings 503, 523 of the carriage 500. The pins 962 now hang in the apertures 505, while the cams 504 engage in the apertures 961. To fix the protective cap 950 in the starting position, the safety cams 125 are locked into the dimples 972.

The feet 204 of the slide rail 200 engage in the depressions 124 in the lower part 100 of the housing. The angled rails 616 of the funnel holder 601 are driven into the vertical grooves 167 of the U-profiles 166 on the upper part 150 of the housing. The funnel 690 sits with its retainer cams 695 and its fixing nose 696 in the notches 619 and in the groove 615, respectively, of the funnel holder 601. The outlet 692 of the funnel 690 with the sealing member 694 is situated in the funnel outlet 608. In addition, the funnel holder 601 is fixed laterally by the limit cams 164 in the upper part 150 of the housing.

The complete counter 700 is held by the supports 159 in the upper part 150 of the housing. The capsule 810 of the valve shield 800 sits to the maximum extent in the valve guide 850, the limit stop flange 851 of the latter sitting in the receiving grooves 104, 156 of the lower part 100 of the housing and the upper part 150 of the housing, respectively, and the connecting web 115 coming into engagement with the notch 856.

### FIGS. 19A to 19D

This sequence of figures illustrates the release of the shutter 400 which surrounds the dosing cavity 302 of the dosing slide 300 filled with medicament, upon swinging the protective cap 950 down.

### FIGS. 19A and 19B

In accordance with Situation A1, the carriage 500 is so positioned that its wings 503, 523 stand before the funnel holder 601, i.e. the pin 962 of the side arm 960 of the protective cap 950, engaging in the aperture 505, is ineffective as regards unlocking the unmovable shutter 400. The blocking cams 609 under the funnel holder 601 engage behind the wings 405 projecting laterally on the shutter 400. The dosing cavity 302 is situated underneath the funnel outlet 608 and could already be filled with medicament.

### FIG. 19C

The protective cap 950 has in the meantime been pulled out completely and the carriage 500 hanging on the side arms 960 has been pulled forward; Situation A2 has been reached. The shutter 400 is still unmovable and, with its closure part 401, surrounds the dosing cavity 302 which has been pushed into the channel inlet 914 of the mouthpiece 900 by means of pulling off the protective cap 950 and filled with medicament.

The swinging-down of the protective cap 950 now commenced, i.e. situation A3 is being implemented. However, the protective cap 950 has not yet been sung down fully, so that the pin 962 ascends in the aperture 505 during the swinging-down movement and consequently gradually raises and unlocks the lamella 605 arresting the shutter 400.

### FIG. 19D

In situation A3 which has been reached--this also applies to Situations A4 to A7--the protective cap 950 has been swung down fully, as a result of which the pin 962 forces the lamella 605 up. The blocking cam 609 is thus disengaged from the wing 405 on the shutter 400. The shutter 400 is movable, i.e. readiness for inhalation exists in conjunction with situation A3. The swung-down protective cap 950 is fixed in this position by the cooperation of the safety cams 125 in the lower part 100 of the housing and the dimples 971 on the side arms 960.

### FIGS. 20A to 20F

This sequence of figures illustrates a complete inhalation cycle with the mechanical events occurring in the different possible situations.

### FIG. 20A

In situation A1 the valve shield 800, the carriage 500 and the shutter 400 are located in their rear end position. This is the state of the inhaler after the protective cap 950 is closed following a correctly performed inhalation or prior to the first use. With the protective cap 950 being pushed in, the shutter 400, the valve shield 800 and the dosing slide 300 have been pushed back into the rear end position by the carriage 500. The pull catch 521 of the carriage 500 grips the carrier 409 of the shutter 400. With its spring wedges 514, the carriage 500 presses against the catches 321 of the dosing slide 300, the spring wedges 514 being enclosed to the inside by the locking teeth 824.

The two struts 508, 510 of the carriage 500 have pushed the valve shield 800 into its starting position. A cam 703 on the units wheel 701 of the counter 700 has been put forward one unit by the cam 306 on the dosing slide 300. The dosing cavity 302 is now once again situated underneath the funnel outlet 608.

### FIG. 20B

In situation A4 the inhaler is in a state of readiness for inhalation. By pulling the protective cap 950 out, the valve shield 800 is advanced from the rearmost position. The carriers 821 on the tentacles 820 have been gripped by the wings 503, 523 and pulled forward slightly, so that the stubs 813 of the valve shield 800 are removed from the holes 855 of the valve guide 850 and create air gaps. The inhaling patient is able to draw breath through these air gaps if no other air inlets are provided on the inhaler. On pulling the protective cap 950 out, the carriage 500 was moved with its grate section 515 past the grate section 621 of the funnel holder, so that a vibration was generated for promoting the flow of the medicament powder from the funnel 690 into the dosing cavity 302. The grate sections 515, 621 are dimensioned and arranged in such a way that when pulling the protective cap 950 out, vibrations are generated only so long as the dosing cavity 302 is situated under the funnel outlet 608. When the carriage 500 begins to pull the dosing slide 300 with it, the grate sections 515 and 621 disengage.

The dosing slide 300 was furthermore gripped via the transverse cams 309 by the pull cams 512 of the carriage 500 and moved forwards in the direction of the mouthpiece 900 to such an extent that the dosing cavity 302 is now surrounded by the closure part 401 of the shutter 400. The shutter 400 is also released, since the blocking cams 609 underneath the funnel holder 601 have lifted from the wings 405 of the shutter 400 as the protective cap 950 swings down. The wedge profiles 823 of the spring arms 822 of the valve shield 800 stand adjacent to the overspring ribs 157 of the upper part 150 of the housing.

Pressure is exerted from above the spring arms 617 of the funnel holder 601 so that all the components lying below are subjected to a certain amount of surface pressure. This increases the tightness and prevents the escape of medicament powder. After the protective cap 950 has been swung down, the inhaler is in a state of readiness for inhalation, and the easier mobility of the shutter 400 is now desired. When the side arms 960 are swung down, the surface pressure acting from above is in part compensated, as the pin 962 ascending in the aperture 505 presses against the lamellae 605. By means of the oval shape of the cam 504 and the geometry of the aperture 961, the cam 504 has a deliberately greater vertical play in the aperture 961 than its horizontal play. The reduced surface pressure now affords easier mobility of the shutter 400 upon inhalation.

### FIG. 20C

In situation A5--the inhaler is closed again after an omitted inhalation--the valve shield 800 remained in its position, i.e. it was not sucked forwards. When the protective cap 950 is applied, the carriage 500 is pushed back; its spring tongues 513 move away from the catches 321 of the dosing slide 300. The valve shield 800 is again pushed into its rearmost position by the carriage 500; the shutter 400 is locked again. The dosing slide 300 remains, however, with its filled dosing cavity 302 in its forward position; it remains there as a result of suitable friction.

### FIGS. 20D

In situation A6--inhalation was interrupted when incomplete--the valve shield 800 has not yet reached its forward position, as a result of which the shutter 400 was not yet displaced, and the dose of medicament remained enclosed. When the protective cap 950 is applied and the carriage 500 pushed back, the dosing slide 300 remains with its unemptied dosing cavity 302 at the front. The spring tongues 513 of the carriage 500 strike via the spring wedges 514 against the catches 321 and are thus bent inwards. In this way the spring wedges 514 strike against the locking teeth 824 and thus push the valve shield 800 back, until the further pushing back of the valve shield 800 by the two struts 508 and 510 of the carriage 500 takes place.

### FIG. 20E

After incomplete inhalation and reclosing of the inhaler--situation A8--the filled dosing slide 300 stands forward, while the valve shield 800 and carriage 500 are again situated in the rear starting position.

### FIG. 20F

In situation A7--after completed inhalation--the protective cap 950 is swung fully down, as a result of which the pins 962 have lifted the lamellae 605 of the funnel holder 601 and the wings 405 of the shutter 400 have been unlocked. During a correct inhalation, the valve shield 800 has been sucked forwards. The spring arms 822 with the wedge profiles 823 have surmounted the overspring ribs 157 in the upper part 150 of the housing as a result of the valve shield 800 moving forwards. The shutter 400 was pushed into its front end position by the advancing valve shield 800, by which the means the dosing cavity 302 became free and the medicament was inhaled by the patient.

During a correct inhalation the valve shield 800 has been sucked forwards, after its spring arms 822 with the wedge profile 823 have surmounted the overspring ribs 157. It is possible to define the necessary suction effort with the geometry of the wedge profile 823 and of the overspring ribs 157, and with the elasticity of the spring arms 822.

During the reverse movement of the carriage 500, its spring wedges 514 sit clamped between the catches 321 and the locking teeth 824 and cannot therefore escape. As a result, the dosing slide 300 and the valve shield 800 are now pushed back into the starting position--Situation A1--by the spring wedges 514 and the struts 508, 510.

The following account describes the construction of the inhaler; although the sequence of steps described herein need not necessarily correspond with the sequence of assembly in mass manufacture.

The valve guide 850 is fitted into the rear of the lower part 100 of the housing and the valve shield 800 is fitted into the valve guide 850. The mouthpiece 900 projects from the front. The inhaler is equipped with the slide rail 200 placed near the mouthpiece 900 and with the carriage 500 bearing on the valve shield 800. The dosing slide 300 is now placed on the slide rail 200. The shutter 400 is added for further completion. The protective cap 950 with the lateral side arms 960, which are attached to the carriage 500, is now fitted. The completely fitted funnel arrangement 600, which is directed towards the protective cap 950, and the counter 700 are shown here in two views. Finally, the upper part 150 of the housing would have to be fitted.

Further constructional variations can be made to the inhaler which has been described. The following variations are expressly mentioned here:
Instead of the dimples 972 for arresting the protective cap 950 in the pushed-in statesituation A1--an outwardly directed cam could in each case be provided near the entrance to the cap 952 on the upper side of the side arms 960 and engage in slots in the front side 160 of the upper part 150 of the housing in the closed state. In order to detach the protective cap 950 from the mouthpiece 900, the protective cap is to be pressed-in in the area of the lateral grip contours 951, as a result of which the cams come out.

In the mouthpiece 900, near the channel outlet 922, it is possible to provide in the enlarged channel section 928 a three-dimensional surface-profiled wall section with a transverse fluting in order to promote the powder deagglomeration and the deposition of coarser particles ineffective for inhalation.

To hold the two halves of the mouthpiece 900 together, complementary connecting elements could be arranged in each case on the inner cut edges of the two halves--for example, a combination of bores and cams--in order for both halves to be joined together again after possible cleaning and drying.

To embed the funnel arrangement in the inhaler, it might be possible to use a collar made of elastic material which is pushed onto the funnel holder 601.

The pharmacological dry powder stored in the funnel 690 can be in loose form on the one hand. However, pre-dosed units for dispensing are also included, for example as an extruded pellet lane or in pearl chain form. Individually dosed units for dispensing could be arranged in blisters or on tape rolls. It will be appreciated that the medicament reservoir and a device for dividing off the individual doses are to be designed in accordance with each other.

The atomizer path 924 in the mouthpiece 900 is designed as a straight or winding channel in which at least one baffle 925 is arranged, and the latter can be an inwardly projecting lamella, a wall, a flow body or a screen.

Instead of the mechanical counter 700, it is also possible to use a chip with which all relevant data are recorded, such as number of inhalations performed, time of intake, and flow parameters.

The flow control realized at present means of the overspring rib 157 and the spring arms 822 could also be obtained by means of an alterable resistance within the valve guide 850.

To regulate the flow rate, it is advantageous to provide an insert for receiving an optional nozzle within the mouth tube 920, namely at the stat of the atomizer path 924 or mounted upstream of the mouth tube.

For specific changing of the flow resistance in the inhaler during an inhalation, which resistance is obtained by the air gap between the fixed valve guide 850 and the moving capsule 810 of the valve shield 800, it is possible to configure this air gap, effective at the respective position of the valve shield 800, incrementally by means of physical irregularities on the surface of the capsule 810 and/or in the inside of the valve guide 850. For this purpose, consideration may be given, for example, to widening or narrowing physical dimensions on the capsule 810 of the valve shield 800 and in the valve guide 850 or grooves whose cross-section changes along their length.

In the inhaler which has been described, but also in inhalers in general, there is the possibility of recording the inhalations and their flow parameters by means of sensor technology. To measure the parameters, use is made of membrane/bending beam technology or a piezoresistive element in combination with a diaphragm or in combination with the Venturi measurement principle. With IPC logic and sensor technology, open-loop control becomes closed-loop control. This closed-loop control makes it possible to govern an adjustable nozzle via an electronic movement element, which nozzle finally regulates the flow in the inhaler constantly by a resistance change.
For current supply, a dynamo is provided in the inside of the inhaler and generates an electric current when the protective cap 950 is opened or when air flows through the inhaler during the inhalation, this electric current being stored and being used to supply the electronic components.

The electronic components, as a plug-in, re-usable control module, can be removed from the inhaler so that a battery operation is possible. The inhalation data are collected by means of an integrated memory chip and made available to the doctor or pharmacist. Exact monitoring of the dose administration is thus possible. The plug-in modules can be recharged on base units for further use and/or can be programmed so that only the contaminated part of the inhaler is to be discarded.

To better monitor the inhalation procedure, a mechanically and/or electronically generated acoustic and/or optical signal is emitted on completion of a successful or unsuccessful inhalation.

It is also possible to arrange the two complementary grate sections 515, 621 on the one hand on the carriage 500 and on the other hand on the lower part 100 of the housing or on the upper part 150 of the housing. So that vibrations occur only when the protective cap 950 is being pulled out--but not when it is being pushed back--one of the two grate sections 515, 621 can be taken out of operation on each replacement of the protective cap 950, e.g. on a component which is also movable.

The desiccant powder at present accommodated in the funnel lid 680 inside the chamber 681 could also be positioned inside the funnel holder 601.

The outer contours of the inhaler and the internal weight distribution mean that when it is laid on an essentially horizontal and dimensionally stable support, the inhaler always orients itself with the outlet 608 of the funnel holder 601 pointing downwards.

The present account describes a "passive" inhaler which is activated through the application of suction by a patient. It is however to be understood that the principles of the invention apply equally to "active" devices which comprise a pressurised gas supply and which are activated by the release of pressurised gas through the device.

## Claims

1. An inhaler for dispensing a dose of an active agent to the respiratory tract of a patient, which inhaler comprises a housing defined by an upper part (150) and a lower part (100) and a mouthpiece (900) which is attached or is adapted to be attached to said housing; wherein said housing includes storage means (690, 680) for storing an active agent and an airway for delivering said dose from the storage means to the mouthpiece in a stream of air or other gas for administration to a patient;
wherein said upper part and said lower part of the housing are joined together by means of a mechanical fixing (111, 173) and are sealed together by a separate sealing portion which serves to maintain a substantially airtight connection between said upper part and said lower part of the housing,
**characterised in that** the sealing portion comprises a flange (179) provided on one of the upper part or the lower part of the housing, which flange is adapted to fit sealingly into a corresponding slot (116) provided on the other of the upper part or the lower part of the housing.

2. An inhaler as claimed in claim 1, wherein said mechanical fixing comprises at least one snap-fit fixing which comprises a male portion (173) provided on one of the upper part or the lower part of the housing and a co-operating female portion (111) provided on the other of the upper part or the lower part of the housing.

3. An inhaler as claimed in claim 2, which comprises a plurality of snap-fit fixings spaced around said housing.

4. An inhaler as claimed in claim 2 or claim 3, wherein the male portion of each snap-fit fixing comprises two parallel co-extending arms which are optionally biased away from one another, and the female portion comprises a recess into which said arms are adapted to be inserted, the arrangement being such that friction created by engagement of the arms with the walls of said recess acts to retain the arms within the recess.

5. An inhaler as claimed in any preceding claim, wherein said active agent is a dry powdered substance.

6. An inhaler as claimed in any preceding claim, further comprising an inlet for drawing air into said airway when suction is applied to said mouthpiece, such as to draw a dose of said active agent from the reservoir to the mouthpiece for administration to the patient.

7. An inhaler as claimed in any preceding claim, further comprising a source of pressurised air or other gas which is adapted on activation to blow a dose of said active agent from the reservoir to the mouthpiece for administration to the patient.

8. An inhaler as claimed in any preceding claim, wherein the sealing action of said sealing portion is such that a gas flow rate of no greater than 45 litres/minute, preferably no greater than 40 litres/minute, is required in order to actuate the inhaler and release the dose of active agent into the airway for administration to the patient.

9. An inhaler as claimed in any preceding claim, wherein the sealing action of said sealing portion is such that a gas flow rate in the range of about 30 to about 40 litres/minute is required in order to actuate the inhaler and release the dose of active agent into the airway for administration to the patient.

10. An inhaler according to claim 1, for multiple dosed administration of a pharmacological dry powder, wherein the mouthpiece includes a channel extending therethrough,
wherein the storage means comprises a medicament reservoir located inside said housing, said medicament reservoir suitable for containing a dry powder and including a funnel outlet,
wherein the inhaler further comprises:
a protective cap covering said mouthpiece, said protective cap being movable between a closed position and an open position;
a movable shutter positioned inside said housing, said shutter including a closure part which protrudes into said channel of said mouthpiece;
a movable dosing slide located inside said housing, said dosing slide being movable between a receiving position and a dispensing position, said dosing slide including a dosing cavity positioned underneath said funnel outlet of said medicament reservoir when said dosing slide is in said receiving position, said dosing cavity being movable into said dispensing position upon moving said protective cap from said closed position to said open position, said dosing cavity moving into said closure part and being surrounded by said closure part upon movement of said dosing slide into said dispensing position;
a movable valve shield positioned inside said housing, said valve shield being movable between a rest position and a forward position in response to suction generated during inhalation, said valve shield moving said shutter when said valve shield moves into said forward position, the movement of said shutter releasing said dosing cavity from said closure part to thereby permit the release of dry powder from said dosing cavity into said channel of said mouthpiece and to permit the inhalation of dry powder by a user of said inhaler;
locking means for locking said valve shield in said forward position only upon a defined minimum intensity of inhalation;
return means for returning said dosing slide to said receiving position only after a correctly completed inhalation, thereby returning said dosing cavity underneath said funnel outlet; and
a recording unit positioned inside said housing, said recording unit recording the number of correctly performed inhalations and having a protrusion that moves from an initial position to a final position such that, when said protrusion reaches its final position, the protrusion obstructs the return means from moving said dosing slide into its receiving position, thereby preventing further use of the inhaler.

11. An inhaler according to claim 1 for multiple dosed administration of a pharmacological dry powder, wherein the storage means comprises a medicament reservoir suitable for containing a dry powder in loose form or in pre-dosed units for dispensing, wherein the mouthpiece is covered by a removable protective cap, wherein the inhaler further comprises a movable dosing slide with a dosing cavity which can be positioned for filling in a starting position underneath a funnel outlet of the medicament reservoir; the arrangement being such that on partial opening of the protective cap, said powder is dosed into the dosing cavity; and on continued opening of the protective cap, the filled dosing cavity is transferred into a channel out of which a patient may inhale said dose of medicament; wherein a movable shutter and a movable valve shield are provided in the housing, which shutter is arranged to close the dosing cavity upon transfer of the dosing cavity into said channel; said valve shield being movable from its resting position on application of suction generated by inhalation, the arrangement being such that on application of a defined minimum level of suction, the valve shield is displaced and comes into contact with said shutter, whereby the shutter is caused to be displaced against adjustable locking means, thereby opening the dosing cavity such that a dose of powder is released therefrom for inhalation by a patient; and wherein means are provided for returning the dosing slide to the filling position with the dosing cavity under the funnel outlet only after correct completion of an inhalation; and wherein an integrated mechanical and/or electronic recording unit is provided, by means of which at least the correctly performed inhalations are recorded; which recording unit is arranged to effect a blocking of the inhaler after a defined number of inhalation doses have been used up.

12. A kit of parts for assembling an inhaler according to any of the preceding claims, wherein the kit comprises a mouthpiece, an upper part of a housing and a lower part of a housing, wherein the upper part and lower part of the housing are joined together by means of a mechanical fixing and are sealed together by a separate sealing portion which comprises a flange (179) provided on one of the upper part or the lower part of the housing, which flange is adapted to fit sealingly into a corresponding slot (116) provided on the other of the upper part or the lower part of the housing.

## Patentansprüche

1. Inhalator zum Verteilen einer Dosis eines aktiven Wirkstoffs an den Respirationstrakt eines Patienten, wobei der Inhalator ein Gehäuse, welches durch ein oberes Teil (150) und ein unteres Teil (100) definiert ist, und ein Mundstück (900), welches an dem Gehäuse angebracht ist oder daran angepasst ist, an dem Gehäuse angebracht zu werden, beinhaltet, wobei das Gehäuse ein Speichermittel (609, 680) zum Speichern eines aktiven Wirkstoffs und einen Luftweg zum Liefern der Dosis in einem Strom von Luft oder eines anderen Gases von dem Speichermittel zu dem Mundstück für ein Verabreichen zu einem Patienten einschließt,
wobei das obere Teil und das untere Teil des Gehäuses mittels einer mechanischen Fixierung (111, 173) aneinander gefügt sind und durch einen separaten Dichtungsabschnitt zusammen gedichtet werden, welcher dazu dient, eine im Wesentlichen luftdichte Verbindung zwischen dem oberen Teil und dem unteren Teil des Gehäuses beizubehalten,
**dadurch gekennzeichnet, dass** der Dichtungsabschnitt einen Flansch (179) beinhaltet, welcher an einem der Teile oberes Teil oder unteres Teil des Gehäuses vorgesehen ist, wobei der Flansch daran angepasst ist, dichtend in einen entsprechenden Spalt (116) zu passen, welcher an dem anderen der Teile oberes Teil oder unteres Teil des Gehäuses vorgesehen ist.

2. Inhalator nach Anspruch 1, wobei die mechanische Fixierung zumindest eine Schnapppassungsfixierung beinhaltet, welche einen männlichen Abschnitt (173), welcher an einem der Teile oberes Teil oder unteres Teil des Gehäuses vorgesehen ist, und einen zusammenwirkenden weiblichen Abschnitt (111), welcher an dem anderen der Teile oberes Teil oder unteres Teil des Gehäuses vorgesehen ist, beinhaltet.

3. Inhalator nach Anspruch 2, welcher eine Vielzahl von Schnapppassungsfixierungen beinhaltet, welche um das Gehäuse herum verteilt sind.

4. Inhalator nach Anspruch 2 oder 3, wobei der männliche Abschnitt jeder Schnapppassungsfixierung zwei sich parallel miteinander erstreckende Arme beinhaltet, welche optional voneinander weg vorgespannt sind, und der weibliche Abschnitt eine Ausnehmung beinhaltet, wobei die Arme angepasst sind, in die Ausnehmung eingeführt zu werden, wobei die Anordnung eine solche ist, dass eine Reibung, welche durch eine Wirkverbindung der Arme mit den Wänden der Ausnehmung erzeugt wird, zum Zurückhalten der Arme in der Ausnehmung wirkt.

5. Inhalator nach einem der voran stehenden Ansprüche, wobei der aktive Wirkstoff eine trockene, pulverige Substanz ist.

6. Inhalator nach einem der voran stehenden Ansprüche, welcher ferner einen Einlass zum Ziehen von Luft in den Luftweg, wenn ein Sog auf das Mundstück aufgebracht wird, so dass eine Dosis des aktiven Wirkstoffs für eine Verabreichung an den Patienten von dem Reservoir zu dem Mundstück gezogen wird, beinhaltet.

7. Inhalator nach einem der voran stehenden Ansprüche, welcher fernen eine Quelle einer mit Druck beaufschlagter Luft oder eines anderen mit Druck beaufschlagten Gases beinhaltet, welche daran angepasst ist, bei einer Aktivierung eines Dosis des aktiven Wirkstoffs für eine Verabreichung an den Patienten von dem Reservoir zu dem Mundstück zu blasen.

8. Inhalator nach einem der voran stehenden Ansprüche, wobei die Dichtwirkung des Dichtungsabschnitts eine solche ist, dass eine Gas-Strömungsrate, die nicht größer als 45 Liter/Minute ist, und die vorzugsweise nicht größer als 40 Liter/Minute ist, nötig ist, um den Inhalator zu betätigen und die Dosis des aktiven Wirkstoffs für eine Verabreichung an den Patienten in den Luftweg freizugeben.

9. Inhalator nach einem der voran stehenden Ansprüche, wobei die Dichtwirkung des Dichtungsabschnitts eine solche ist, dass eine Gas-Strömungsrate in dem Bereich von ungefähr 30 Liter/Minute bis ungefähr 40 Liter/Minute nötig ist, um den Inhalator zu betätigen und die Dosis des aktiven Wirkstoffs für eine Verabreichung an den Patienten in den Luftweg freizugeben.

10. Inhalator nach Anspruch 1 für viele dosierte Verabreichungen eines pharmakologischen trockenen Pulvers, wobei das Mundstück einen Kanal einschließt, welcher sich da hindurch erstreckt,
wobei das Speichermittel ein Medikamentenreservoir beinhaltet, welche innerhalb des Gehäuses angeordnet ist, wobei das Medikamentenreservoir dazu geeignet ist, ein trockenes Pulver aufzubewahren, und einen trichterartigen Auslass einschließt,
wobei der Inhalator ferner beinhaltet:
eine Schutzkappe, welche das Mundstück bedeckt, wobei die Schutzkappe zischen einer geschlossenen Position und einer geöffneten Position beweglich ist,
eine bewegliche Blende, welche innerhalb des Gehäuses positioniert ist, wobei die Blende einen Verschlussteil einschließt, welcher in den Kanal des Mudstückes hinein kragt,
einen beweglichen Dosierschieber, welcher innerhalb des Gehäuses angeordnet ist, wobei der Dosierschieber zwischen einer Aufnahmeposition und einer Verteilposition beweglich ist, wobei der Dosierschieber eine Dosierkavität einschließt, welche unter dem trichterartigen Auslass des Medikamentenreservoirs positioniert ist, wenn sich der Dosierschieber in der Aufnahmeposition befindet, wobei die Dosierkavität auf ein Bewegen der Schutzkappe von der geschlossenen Position zu der geöffneten Position hin in die Verteilposition hin beweglich ist, wobei sich die Dosierkavität auf eine Bewegung des Dosierschiebers in die Verteilposition hin in das Verschlussteil hinein bewegt und von dem Verschlussteil umfangen wird,
ein bewegliches Ventilschild, welches innerhalb des Gehäuses positioniert ist, wobei das Ventilschild im Ansprechen auf einen während einer Inhalation erzeugten Sog zwischen einer Ruheposition und einer vorwärtigen Position beweglich ist, wobei das Ventilschild die Blende bewegt, wenn sich das Ventilschild in die vorwärtige Position bewegt, wobei die Bewegung der Blende die Dosierkavität von dem Verschlussteil freigibt, um dadurch die Freigabe von trockenem Pulver von der Dosierkavität in den Kanal des Mundstückes zu gestatten, und um die Inhalation eines trockenen Pulvers durch einen Verwender des Inhalator zu gestatten,
Sperrmittel, um das Ventilschild in der vorwärtigen Position nur auf eine definierte minimale Intensität einer Inhalation hin zu sperren,
Rückleitmittel, um den Dosierschieber nur nach einer korrekt abgeschlossenen Inhalation in die Aufnahmeposition zurück zu leiten, wodurch die Dosierkavität unter den trichterartigen Auslass zurück geleitet wird, und
eine Aufzeichnungseinheit, welche innerhalb des Gehäuses positioniert ist, wobei die Aufzeichnungseinheit die Anzahl korrekt durchgeführter Inhalationen aufzeichnet und eine Auskragung hat, welche sich von einer Anfangsposition zu einer Endposition bewegt, so dass die Auskragung die Rückleitmittel an einem Bewegen des Dosierschiebers in dessen Aufnahmeposition hindert, wenn die Auskragung deren Endposition erreicht, wodurch eine weitere Verwendung des Inhalators verhindert wird.

11. Inhalator nach Anspruch 1 für viele dosierte Verabreichungen eines pharmakologischen trockenen Pulvers, wobei das Speichermittel ein Medikamentenreservoir beinhaltet, welches dazu geeignet ist, ein trockenes Pulver in loser Form oder in vordosierten Einheiten zum Verteilen aufzubewahren, wobei das Mundstück durch eine entfernbare Schutzkappe bedeckt ist, wobei der Inhalator ferner beinhaltet:
einen beweglichen Dosierschieber mit einer Dosierkavität, welche zum Füllen in eine Startposition unter einem trichterartigen Auslass des Medikamentenreservoirs positioniert werden kann, wobei die Anordnung eine solche ist, dass bei einem teilweisen Öffnen der Schutzkappe das Pulver in die Dosierkavität dosiert wird, und bei einem fortgesetzten öffnen der Schutzkappe die gefüllte Dosierkavität in einen Kanal transferiert wird, aus welchem heraus ein Patient die Dosis an Medikament inhalieren kann, wobei eine bewegliche Blende und ein bewegliches Ventilschild in dem Gehäuse vorgesehen sind, wobei die Blende so angeordnet ist, dass diese die Dosierkavität auf einen Transfer der Dosierkavität in den Kanal hin schließt, wobei der Ventilschild von dessen Ruheposition bei einem Aufbringen eines durch eine Inhalation erzeugten Soges beweglich ist, wobei die Anordnung eine solche ist, dass bei einem Aufbringen eines definierten minimalen Levels an Sog das Ventilschild verlagert wird und mit der Blende in Kontakt kommt, wodurch verursacht wird, dass die Blende gegen einstellbare Sperrmittel verlagert wird, wodurch sich die Dosierkavität öffnet, so dass eine Dosis an Pulver davon für eine Inhalation durch einen Patienten freigegeben wird, und wobei Mittel vorgesehen sind, um nur nach einem korrekten Abschluss einer Inhalation den Dosierschieber zu der Füllposition, bei welcher sich die Dosierkavität unter dem trichterartigen Auslass befindet, zurück zu leiten, und wobei eine integrierte mechanische und/oder elektronische Aufzeichnungseinheit vorgesehen ist, mittels welcher zumindest die korrekt durchgeführten Inhalationen aufgezeichnet werden, wobei die Aufzeichnungseinheit angeordnet ist, um ein Blockieren des Inhalators zu bewirken, nachdem eine definierte Anzahl an Inhalationsdosen verbraucht worden sind.

12. Ein Kit von Teilen zum Zusammenbauen eines Inhalators gemäß den voran stehenden Ansprüchen, wobei das Kit ein Mundstück, ein oberes Teil eines Gehäuses und ein unteres Teil eines Gehäuses beinhaltet, wobei das obere Teil und das untere Teil des Gehäuses mittels einer mechanischen Fixierung miteinander verbunden werden und durch einen separaten Dichtabschnitt zusammen dichten, welcher einen Flansch (179) beinhaltet, der an einem der Teile oberes Teil oder unteres Teil des Gehäuses vorgesehen ist, wobei der Flansch daran angepasst ist, dichtend in einen entsprechenden Spalt (116) zu passen, welcher an dem anderen der Teile oberes Teil oder unteres Teil des Gehäuses vorgesehen ist.

## Revendications

1. Inhalateur pour administrer une dose d'agent actif dans les voies respiratoires d'un patient, lequel inhalateur comprend un boîtier qui est défini par une partie supérieure (150) et une partie inférieure (100), et un embout buccal (900) qui est relié ou apte à être relié au boîtier ; étant précisé que le boîtier contient des moyens de stockage (690, 680) pour stocker un agent actif, et un conduit d'air pour amener la dose à l'embout buccal à partir des moyens de stockage, sous la forme d'un flux d'air ou d'un autre gaz en vue de l'administration à un patient ;
étant précisé que la partie supérieure et la partie inférieure du boîtier sont assemblées à l'aide d'une fixation mécanique (111, 173) et sont scellées ensemble par une partie de scellement séparée qui sert à maintenir une liaison globalement étanche à l'air entre la partie supérieure et la partie inférieure du boîtier,
**caractérisé en ce que** la partie de scellement comprend un rebord (179) qui est prévu sur la partie supérieure ou sur la partie inférieure du boîtier, lequel rebord est apte à s'emboîter en vue d'un scellement dans une fente correspondante (116) qui est prévue sur la partie inférieure ou sur la partie supérieure du boîtier.

2. Inhalateur tel qu'il est revendiqué dans la revendication 1, étant précisé que la fixation mécanique comprend au moins une fixation par enclenchement qui comprend une partie mâle (173) prévue sur la partie supérieure ou sur la partie inférieure du boîtier, et une partie femelle coopérante (111) prévue sur la partie inférieure ou sur la partie supérieure du boîtier.

3. Inhalateur tel qu'il est revendiqué dans la revendication 2, qui comprend plusieurs fixations par enclenchement espacées autour du boîtier.

4. Inhalateur tel qu'il est revendiqué dans la revendication 2 ou la revendication 3, étant précisé que la partie mâle de chaque fixation par enclenchement comprend deux bras qui s'étendent parallèlement et qui sont à titre optionnel contraints pour être éloignés l'un de l'autre, et que la partie femelle comprend un creux dans lequel les bras sont aptes à être introduits, la disposition étant telle que le frottement créé par l'accouplement des bras avec les parois du creux agit pour retenir les bras à l'intérieur du creux.

5. Inhalateur tel qu'il est revendiqué dans l'une quelconque des revendications précédentes, étant précisé que l'agent actif est une substance poudreuse sèche.

6. Inhalateur tel qu'il est revendiqué dans l'une quelconque des revendications précédentes, comprenant par ailleurs un orifice d'entrée pour aspirer de l'air dans le conduit d'air quand une aspiration est appliquée sur l'embout buccal, de manière à aspirer une dose d'agent actif, à partir du réservoir, jusque dans ledit embout, en vue d'une administration au patient.

7. Inhalateur tel qu'il est revendiqué dans l'une quelconque des revendications précédentes, comprenant par ailleurs une source d'air ou d'un autre gaz comprimés, qui est apte, lorsqu'elle est activée, à projeter une dose d'agent actif dans l'embout buccal, à partir du réservoir, en vue d'une administration au patient.

8. Inhalateur tel qu'il est revendiqué dans l'une quelconque des revendications précédentes, étant précisé que l'action de scellement de ladite partie de scellement est telle qu'un débit de gaz non supérieur à 45 litres/minute, de préférence non supérieur à 40 litres/minute, est nécessaire pour actionner l'inhalateur et libérer la dose d'agent actif dans le conduit d'air en vue d'une administration au patient.

9. Inhalateur tel qu'il est revendiqué dans l'une quelconque des revendications précédentes, étant précisé que l'action de scellement de ladite partie de scellement est telle qu'un débit de gaz situé dans la plage d'environ 30 à environ 40 litres/minute est nécessaire pour actionner l'inhalateur et libérer la dose d'agent actif dans le conduit d'air en vue d'une administration au patient.

10. Inhalateur tel qu'il est revendiqué dans la revendication 1, pour une administration multidosée d'une poudre sèche pharmacologique, étant précisé que l'embout buccal présente un conduit qui le traverse,
que les moyens de stockage comprennent un réservoir de médicament qui se trouve à l'intérieur du boîtier, le réservoir de médicament étant apte à contenir une poudre sèche et comprenant une sortie d'entonnoir,
que l'inhalateur comprend par ailleurs :
un capuchon de protection qui couvre l'embout buccal, ledit capuchon de protection étant mobile entre une position fermée et une position ouverte ;
un obturateur mobile qui est placé à l'intérieur du boîtier, ledit obturateur comprenant une pièce de fermeture qui fait saillie dans le conduit de l'embout buccal ;
un coulisseau de dosage mobile qui se trouve à l'intérieur du boîtier, ledit coulisseau de dosage étant mobile entre une position de réception et une position de distribution, et présentant une cavité de dosage placée sous la sortie d'entonnoir du réservoir de médicament quand le coulisseau est dans la position de réception, la cavité de dosage étant mobile jusqu'à la position de distribution lorsque le capuchon de protection passe de la position fermée à la position ouverte, ladite cavité de dosage entrant dans la pièce de fermeture et étant entourée par celle-ci lorsque le coulisseau de dosage passe en position de distribution ;
une valve mobile qui est placée à l'intérieur du boîtier, ladite valve étant mobile entre une position de repos et une position avant en réponse à l'aspiration produite pendant l'inhalation, la valve déplaçant l'obturateur quand elle passe à ladite position avant, le déplacement de l'obturateur dégageant la cavité de dosage par rapport à la pièce de fermeture pour permettre ainsi la libération de poudre sèche à partir de ladite cavité de dosage jusque dans le conduit de l'embout buccal, et pour permettre l'inhalation de poudre sèche par un utilisateur de l'inhalateur ;
des moyens de verrouillage pour ne verrouiller la valve dans la position avant qu'en présence d'une intensité minimale définie d'inhalation ;
des moyens de rappel pour ne ramener le coulisseau de dosage à la position de réception qu'après une inhalation réalisée correctement, ramenant ainsi la cavité de dosage sous l'entonnoir de sortie ; et
une unité d'enregistrement qui est placée à l'intérieur du boîtier, l'unité d'enregistrement enregistrant le nombre d'inhalations réalisées correctement, et présentant une saillie qui passe d'une position initiale à une position finale de telle sorte que ladite saillie, lorsqu'elle atteint sa position finale, empêche les moyens de rappel d'amener le coulisseau de dosage dans sa position de réception, empêchant ainsi de continuer à utiliser l'inhalateur.

11. Inhalateur tel qu'il est revendiqué dans la revendication 1, pour une administration multidosée d'une poudre sèche pharmacologique, étant précisé que les moyens de stockage comprennent un réservoir de médicament apte à contenir une poudre sèche sous une forme en vrac ou en unités prédosées pour la distribution, étant précisé que l'embout buccal est couvert par un capuchon de protection amovible, que l'inhalateur comprend par ailleurs un coulisseau de dosage mobile pourvu d'une cavité de dosage qui peut être positionnée pour le remplissage dans une position de départ sous une sortie d'entonnoir du réservoir de médicament ; la disposition étant telle que lors d'une ouverture partielle du capuchon de protection, ladite poudre est dosée dans la cavité de dosage ; et que lors de la poursuite de l'ouverture du capuchon de protection, la cavité de dosage remplie est transférée dans un conduit à partir duquel un patient peut inhaler la dose de médicament ; qu'un obturateur mobile et une valve mobile sont prévus dans le boîtier, lequel obturateur est conçu pour fermer la cavité de dosage lors du transfert de la cavité de dosage dans le conduit ; la valve étant mobile à partir de sa position de repos lors de l'application d'une aspiration produite par l'inhalation, la disposition étant telle que lors de l'application d'un niveau minimal défini d'aspiration, la valve est déplacée et vient en contact avec l'obturateur, ce qui provoque le déplacement de l'obturateur à l'encontre de moyens de verrouillage réglables, ouvrant ainsi la cavité de dosage de telle sorte qu'une dose de poudre est dégagée de celle-ci pour l'inhalation par un patient ; et étant précisé qu'il est prévu des moyens pour ne ramener le coulisseau de dosage dans la position de remplissage, avec la cavité de dosage sous la sortie d'entonnoir, qu'après qu'une inhalation a été réalisée correctement ; et étant précisé qu'il est prévu une unité d'enregistrement mécanique et/ou électronique intégrée grâce à laquelle sont enregistrées au moins les inhalations réalisées correctement ; laquelle unité d'enregistrement est conçue pour provoquer un blocage de l'inhalateur après qu'un nombre défini de doses d'inhalations ont été utilisées.

12. Kit de pièces pour assembler un inhalateur selon l'une quelconque des revendications précédentes, étant précisé que le kit comprend un embout buccal, une partie supérieure d'un boîtier et une partie inférieure d'un boîtier, et que la partie supérieure et la partie inférieure du boîtier sont réunies à l'aide d'une fixation mécanique et sont scellées ensemble par une partie de scellement séparée qui comprend un rebord (179) prévu sur la partie supérieure ou la partie inférieure du boîtier, lequel rebord est apte à s'emboîter en vue d'un scellement dans une fente correspondante (116) qui est prévue sur la partie inférieure ou sur la partie supérieure du boîtier.
